# EUROPEAN PATENT SPECIFICATION

(11) **EP 4 176 267 B1**
(45) Date of publication and mention of the grant of the patent: **31.07.2024**
(21) Application number: 21739251.3
(22) Date of filing: 30.06.2021
(51) Int. Cl.: G01N 33/68, A61P 15/08

(54) **AMH AND/OR INHIBIN B AS A BIOMARKER FOR AN EFFECT OF A TREATMENT TO IMPROVE MALE FERTILITY**
AMH UND/ODER INHIBIN B ALS BIOMARKER FÜR EINE WIRKUNG EINER BEHANDLUNG ZUR VERBESSERUNG DER MÄNNLICHEN FRUCHTBARKEIT
AMH ET/OU INHIBIN B COMME BIOMARQUEUR DE L' EFFET D'UN TRAITEMENT POUR AMÉLIORER LA FERTILITÉ MASCULINE

(30) Priority: 03.07.2020 DK PA202070456
(43) Date of publication of application: 10.05.2023
(73) Proprietor: XY Therapeutics ApS, 2920 Charlottenlund (DK)
(72) Inventor: JENSEN, Martin, Blomberg, 2920 Charlottenlund (DK)
(74) Representative: Plougmann Vingtoft a/s
(86) International application number: PCT/DK2021/050218
(87) International publication number: WO 2022/002336

(56) References cited:
- WO-A1-2015/018421
- WO-A1-2016/112916
- JENSEN MARTIN BLOMBERG ET AL: "RANKL is a novel regulator of male fertility", ANNE JØRGENSEN, CHRISTINE HJORT ANDREASSEN, IDA BOISEN, PETER SCHWARZ, ANDERS JUUL, ROLAND BARON, BEATE LANSKE 2019 ANNUAL MEETING OF THE AMERICAN SOCIETY FOR BONE AND MINERAL RESEARCH, vol. 34, 1 December 2019 (2019-12-01), pages 193 - 194, XP055788636
- BLOMBERG JENSEN MARTIN ET AL: "RANKL regulates male reproductive function", NATURE COMMUNICATIONS, vol. 12, no. 1, 23 April 2021 (2021-04-23), XP055842471, Retrieved from the Internet <URL:https://www.nature.com/articles/s41467-021-22734-8.pdf> DOI: 10.1038/s41467-021-22734-8

## Description

### Technical field of the invention

The present invention relates to AMH and/or inhibin B as a biomarker for an effect of a treatment to improve fertility in a male subject.

### Background of the invention

Decreased semen quality is a major factor of male infertility. Semen quality is a measure of the ability of the semen to accomplish fertilization. Evaluation of male fertility potential is today determined by semen analysis. A semen analysis evaluates certain characteristics of a male's semen and the spermatozoa contained in the semen. The characteristics measured by the current tests for semen analysis are only some of the clinical important factors for semen quality. The most common variables measured to evaluate sperm quality are: sperm count, motility and morphology. Other variables are volume, DNA fragmentation index, fructose level and pH.

WO 2011/137906 discloses a method for predicting the fertility potential in a male mammal. In particular, it relates to a method for predicting the fertility potential in a male mammal by determining the expression of at least one protein of the vitamin D metabolising machinery in a semen sample.

EP3244911 B1 discloses a method for determining a likely effect of a treatment to improve male fertility, the method comprises determining the level of OPG in a blood serum sample.

WO 2015/018421 discloses RANKL targeting antibodies for use in the treatment of male infertility.

In particular, it would be of great benefit to have a test, which could predict whether a male subject would benefit from a treatment to improve male fertility. Thus, developing a marker that can guide the doctor in this choice would be a clear advantage.

### Summary of the invention

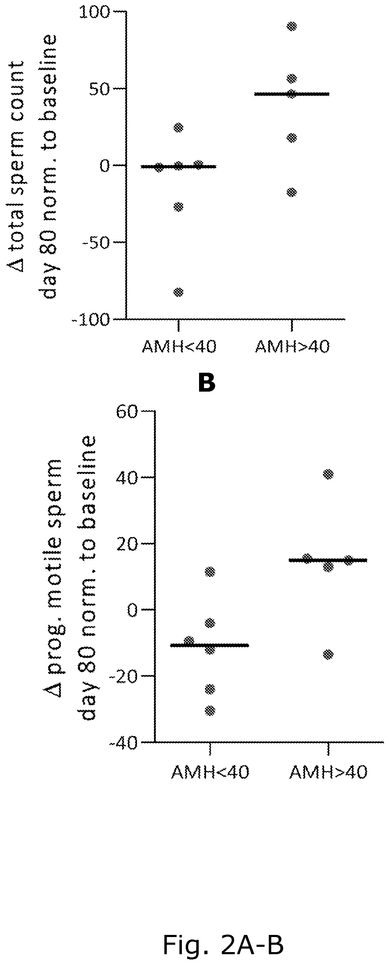

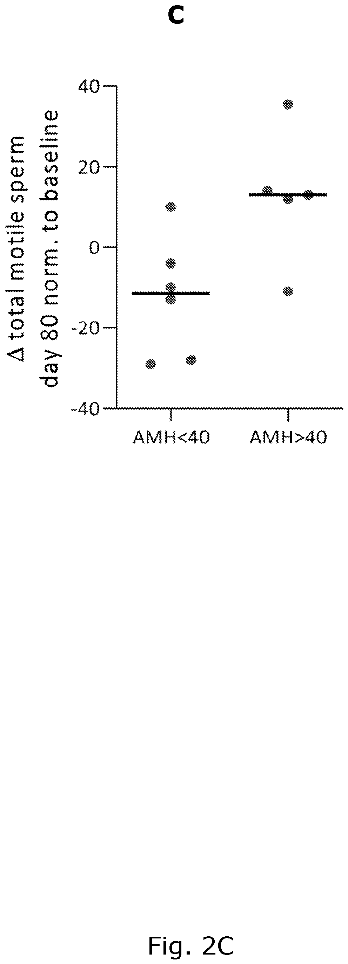

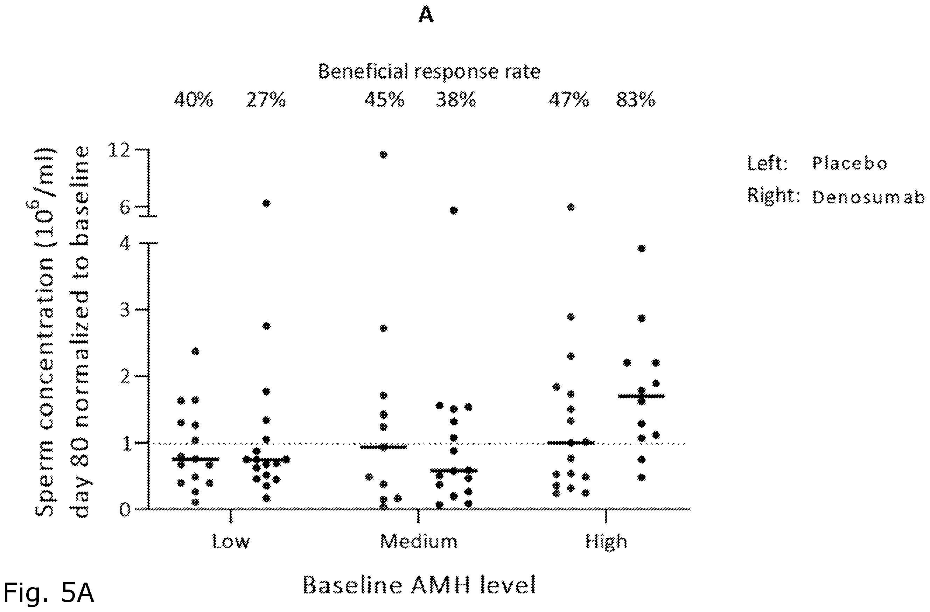

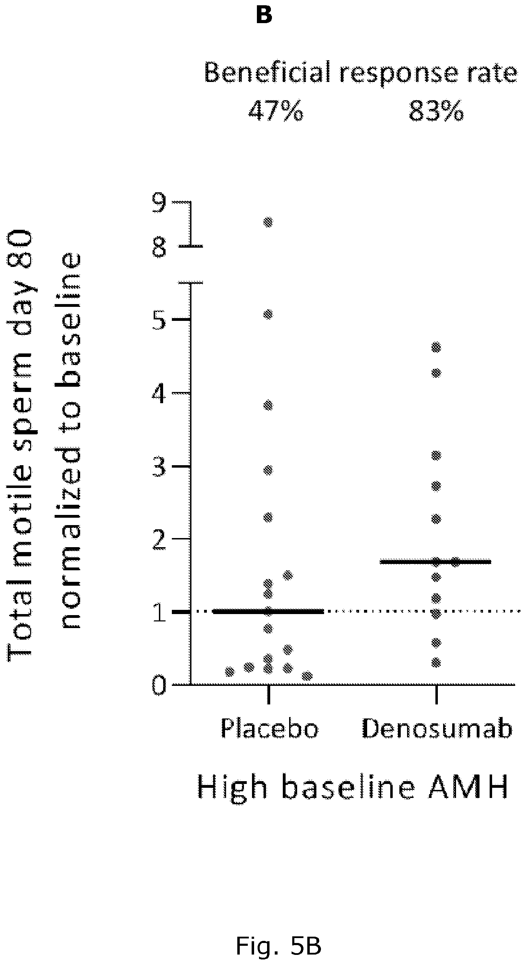

The present invention relates to the identification of AMH and/or inhibin B as a strong biomarker to predict whether a male subject would benefit from a treatment to improve male fertility, in relation to treatment using an antagonist or inhibitor of RANKL (see e.g. example 1 and figures 2 + 5). The predictive value can be further increased when including other biomarkers or ratio of biomarkers.

Thus, an object of the present invention relates to provision of improved biomarkers, which may assist in determining whether a male subject would benefit from a treatment to improve male fertility.

Thus, one aspect of the invention relates to a method for determining a likely effect of a treatment to improve male fertility, the method comprising
- determining the level of AMH and/or inhibin B in a biological sample from a male subject;
- comparing said determined AMH level to a corresponding reference level; and
- determining that said subject will
   ∘ likely benefit from treatment with an antagonist or inhibitor of RANKL, if said level is equal to or higher than said corresponding reference level; or
   ∘ likely not benefit from treatment with an antagonist or inhibitor of RANKL, if said level is lower than said corresponding reference level.

Another aspect of the present invention relates to the use of AMH and/or inhibin B as a biomarker for determining a likely effect of a treatment to improve male fertility.

Yet another aspect of the present invention is to provide a composition comprising an antagonist or inhibitor of RANKL for use in the treatment of male infertility in subject, wherein said subject has a level of AMH of:
- above 30 pmol/l in blood serum, such as at least 35 pmol/l AMH or such as at least 40 pmol/l AMH; and/or
- above 5 pmol/l AMH in seminal fluid.

### Brief description of the figures

Figure 1 shows an overview of the clinical study (RCT) protocol conducted.
Figure 2 shows levels of serum AMH is a predictive marker for effect of RANKL inhibition on male fertility. **A)** Delta total sperm count. **B)** Delta prog. motile sperm. **C)** Delta motile sperm.
Figure 3 shows spontaneous pregnancies. Denosumab treatment induced significantly more spontaneous pregnancies in oligospermic men compared with placebo.
Figure 4 shows pregnancies for men with serum AMH >30. Denosumab treatment induced significantly more spontaneous pregnancies in men with serum AMH > 30 pmol/l.
Figure 5 shows subgroup analysis based on AMH plasma levels: **A)** Sperm concentration. **B)** Total motile sperm. Denosumab treatment of men with the highest serum AMH (tertile) higher sperm concentration and 83% had a beneficial response.
Figure 6 shows subgroup analysis based on serum AMH >35 + top 50% of serum RANKL. **A)** Sperm concentration. **B)** Total sperm count. **C)** Total motile sperm. **D)** Progressive sperm motility. **E)** Morphological normal sperm. Denosumab treatment of men with serum AMH > 35 pmol/l and top 50% of serum RANKL resulted in significantly higher sperm concentration and total motile sperm compared with placebo - beneficial response in 90-100%.
Figure 7 shows subgroup analysis based OPG/sRANKL ratio. If you expect 100-600 percent increase in sperm production within 80 days a high RANKL activity defined by low seminal OPG/RANKL ratio, or vice versa, high seminal RANKL/OPG ratio is beneficial.
Figure 8 shows subgroup analysis based on RANKL (low seminal OPG/RANKL ratio) and AMH. **A)** Sperm concentration. **B)** Total sperm count. **C)**. Progressive sperm motility **D)** Total motile sperm. **E)** Morphological normal sperm. Significant increase in sperm production within 80 days in men with high AMH and high RANKL activity defined by low seminal OPG/RANKL ratio or vice versa high seminal RANKL/OPG ratio.
Figure 9 shows AMH/inhibin B ratio upper quatile and serum RANKL/OPG ratio highest 50%.
Figure 10 shows changes in semen quality over time after Denosumab (60 mg) injection in infertile men stratified after Inhibin B in serum prior to start of treatment and treatment response evaluated by percentual change in total motile sperm/total progressive motile sperm at day 80. Baseline is calculated as average of two semen samples delivered prior to treatment start.
Figure 11 shows changes in sperm concentration over time after Denosumab (60 mg) injection in infertile men stratified after serum levels of **A)** RANKL; **B)** Testosterone ; **C)** Phosphate; **D)** AMH; **E)** AMH/Testosterone ratio; and **F)** AMB/Inhibin B ratio prior to start of treatment.
Figure 12 shows changes in sperm concentration over time after Denosumab (60 mg) injection in infertile men stratified after semen fluid levels of **A)** AMH, (R2 Linear=0.004; Y=-0.16-4.22E-3*x); **B)** inhibin B; and **C)** OPG:RANKL ratio, and **D)** OPG prior to start of treatment.

The present invention will now be described in more detail in the following.

### Detailed description of the invention

### Definitions

Prior to discussing the present invention in further details, the following terms and conventions will first be defined:

### Oligospermia

In the present context, the term "oligospermia" or "oligozoospermia" or "low sperm count" refer to semen with a low concentration of sperm and is a common finding in male infertility. Often semen with a decreased sperm concentration may also show significant abnormalities in sperm morphology and motility (technically oligoasthenoteratozoospermia). There has been interest in replacing the descriptive terms used in semen analysis with more quantitative information.

### Anti-Mü/lerian hormone (AMH)

Anti-Müllerian hormone (AMH), also known as Müllerian-inhibiting hormone (MIH), is a glycoprotein hormone structurally related to inhibin and activin from the transforming growth factor beta superfamily.

### RANKL

Receptor activator of nuclear factor kappa-B ligand (RANKL), also known as tumor necrosis factor ligand superfamily member 11 (TNFSF11), TNF-related activation-induced cytokine (TRANCE), osteoprotegerin ligand (OPGL), and osteoclast differentiation factor (ODF), is a protein that in humans is encoded by the TNFSF11 gene.

RANKL is known as a type II membrane protein and is a member of the tumor necrosis factor (TNF) superfamily. RANKL has been identified to affect the immune system and control bone regeneration and remodeling. RANKL is an apoptosis regulator gene, a binding partner of osteoprotegerin (OPG), a ligand for the receptor RANK and controls cell proliferation by modifying protein levels of Id4, Id2 and cyclin D1.

RANKL is expressed in several tissues and organs including: skeletal muscle, thymus, liver, colon, small intestine, adrenal gland, osteoblast, mammary gland epithelial cells, prostate and pancreas. Variation in concentration levels of RANKL throughout several organs reconfirms the importance of RANKL in tissue growth (particularly bone growth) and immune functions within the body.

Studies in human testis and vitamin D receptor *(VDR)* knock-out mice have shown that several bone factors such as Runx2, osterix, and osteocalcin are expressed in the normal testis and testicular cancer. One of these factors is the receptor activator of NF-,cB ligand (RANKL). The RANKL system is a powerful regulator of bone resorption that comprises three components: RANKL, a transmembrane ligand that following binding to the receptor RANK on a neighbouring cell subsequently activates NF-κB and regulates cell cycle i.e. proliferation, differentiation and apoptosis. The transmembrane RANKL protein resides in osteocytes and activates RANK in immature osteoclasts, which induces osteoclastogenesis and promotes bone resorption. Osteoprotegerin (OPG) is an endogenous secreted decoy receptor that binds RANKL and blocks its signaling thereby preventing osteoclast differentiation and activation. RANKL can also be found in circulation, suggesting a putative endocrine function of the protein. Indeed, recently novel extra-skeletal functions of RANKL have been proposed including regulation of glucose homeostasis.

In the human seminiferous tubules, a marked transcriptional expression of *RANKL, RANK* and *OPG* was found). All three isoforms of RANKL are expressed in the human testis and both the transmembrane and soluble forms of RANKL are expressed at moderate to high levels. By using antibodies targeting transmembrane or extracellular domain, respectively expression of RANKL in adult Sertoli cells and in male germ cells was found. The level of RANKL expression varied within samples but was detected in the majority of Sertoli cells and in spermatocytes and spermatids.

In the present context, the terms "soluble RANKL" or "sRANKL" refer to the free fraction of RANKL. sRANKL is not bound to OPG.

### RANK

Receptor activator of nuclear factor κ B (RANK), also known as TRANCE receptor or TNFRSF11A, is a member of the tumor necrosis factor receptor (TNFR) molecular sub-family. RANK is the receptor for RANK-Ligand (RANKL) and part of the RANK/RANKL/OPG signaling pathway that regulates osteoclast differentiation and activation. It is associated with bone remodeling and repair, immune cell function, lymph node development, thermal regulation, and mammary gland development. Osteoprotegerin (OPG) is a decoy receptor for RANK, and regulates the stimulation of the RANK signaling pathway by competing for RANKL. The cytoplasmic domain of RANK binds TRAFs 1, 2, 3, 5, and 6 which transmit signals to downstream targets such as NF-κB and JNK.

In mice, RANKL, RANK and OPG are detected at transcriptional and protein level in the testis at 16-18 weeks of age. RANKL is expressed in the cytoplasm/membrane of mature Sertoli cells and in spermatocytes and spermatids. RANK is exclusively expressed in the cytoplasm of germ cells with the most prominent expression in spermatogonia. OPG is expressed in the cytoplasm of peritubular cells, the junction between Sertoli and spermatogonia and in spermatids.

### OPG

Osteoprotegerin (OPG), also known as osteoclastogenesis inhibitory factor (OCIF) or tumour necrosis factor receptor superfamily member 11B (TNFRSF11B), is a cytokine receptor of the tumour necrosis factor (TNF) receptor superfamily encoded by the TNFRSF11B gene.

In humans, RANK is exclusively expressed in the cytoplasm/membrane of the germ cells with the most prominent expression in spermatogonia, while OPG is expressed mainly in the cytoplasm of peritubular cells or on the border of the spermatogonia and in spermatids. The soluble isoform of RANKL (sRANKL, observed at 27-31 kDa) is expressed at a higher level than the transmembrane isoform (45 kDa). Both forms of RANKL were together with RANK and OPG robustly expressed in samples with normal spermatogenesis.

### Fertility/fertility potential

In the present context, if a person has a high sperm number (above 40 millions), above 50% motile sperm and more than 12 % morphological normal spermatozoa, the person is regarded to have a normal fertility potential.

In the present context, if a person has a low sperm number (below 40 millions), below 50% motile sperm or less than 12 % morphological normal spermatozoa the person is regarded to unlikely to have a normal fertility potential.

The skilled person would know how to perform such analysis and set a different cut-off between when a male subject is considered to have normal fertility.

This is not an accurate estimate of male fertility potential and the result of the semen analysis does not always correlate with the ability to have children. This is also illustrated by the fact that WHO has recently changed the reference levels as a large proportion of the male population otherwise would be regarded as having a fertility potential below normal. No precise method for predicting the male fertility potential exists presently.

More than 9 % of all babies are born after assisted reproductive methods in Denmark, and the incidence of infertility is increasing worldwide. About 50% of cases are attributable to the male partner and the first step in the clinical evaluation involves semen analysis to evaluate male fertility potential. The clinicians use the analysis in deciding the appropriate reproductive method.

It is estimated that some 1.400.000 semen analyses are performed each year worldwide.

Semen analysis is currently a cumbersome and lengthy process. It is performed by standard microscopy and is based on the subjective analysis of three key parameters: motility, morphology, and total sperm number. The analysis has several drawbacks as it necessitates a trained medical staff, presence of the patient with a fresh semen sample (less than 1 hour old), laboratory facilities etc. making it a costly and time consuming procedure. Logistically, the analysis requires the patient to deliver a semen sample at the laboratory where the testing is performed, which for many patients is considered a major embarrassment. Furthermore, semen analyses using the current methods is highly prone to intra- and inter observer variability due to the subjective assessment of the key parameters, which severely can affect the utility of the analysis, whereby the clinician is troubled in the guidance of the couple to the relevant assisted reproductive method.

Due to above mentioned problems with the currently used methods, a large need exist for a new method for semen analysis, which accurately and objectively can assess male fertility potential in order to stratify patients into the correct reproductive method. The problems with the existing analysis are illustrated by the latest WHO recommendations from 2010, where the reference values for normal semen analysis were changed and caused a huge debate in fertility and andrology meetings worldwide.

### Male subject

Reference to a "male subject" or "subject" or an "individual" includes a human or non-human species of mammals including primates, livestock animals (e.g. sheep, cows, pigs, horses, donkey, goats), laboratory test animals (e.g. mice, rats, rabbits, guinea pigs, hamsters) and companion animals (e.g. dogs, cats). The present invention has applicability, therefore, in human medicine as well as having livestock and veterinary and wild life applications. In a preferred embodiment, the mammal is a human. In a particular preferred embodiment, the mammal is a man.

### Semen sample

Semen is a mixture of an excreted fluid and cells. The fluid is also known as seminal fluid that usually contains spermatozoa. It is secreted by the gonads (sexual glands) and other sexual organs of male or hermaphroditic animals and may be able to fertilize female ova. In humans, seminal fluid may contain several components besides spermatozoa: proteolytic and other enzymes as well as fructose are elements of seminal fluid which promote the survival of spermatozoa and provide a medium through which they mature and get the ability to move or "swim". The process that results in the discharge of semen is called ejaculation.

In a preferred embodiment, the semen sample is to be understood as a sample comprising semen and/or components derived from semen.

In a preferred embodiment, the semen sample is a sample comprising spermatozoa. Rarely in the clinic subjects are seen with semen samples comprising very few or even no spermatozoa in their semen, samples from such a subjects is still of interest and thus included by the present definition of the sample base, as such samples would indeed be a sample which would be indicative of low fertility potential.

The semen sample may be obtained after ejaculation, aspiration from the testis, epididymis or after testicular biopsy or microdissection of the testis.

In the most preferred embodiment, the semen sample is obtained after ejaculation.

In a particular preferred embodiment, the semen sample is a sample comprising spermatozoa and/or components derived from an ejaculate.

Procedures for collecting semen samples from human or animals such as farm animals is well described in the literature and well known for a person skilled in the art.

In another embodiment of the present invention, a minimum of handling steps of the sample is necessary before measuring the expression level(s).

In the present context, the subject "handling steps" relates to any kind of pre-treatment of the semen sample before determining the expression level(s) of the at least one protein of the vitamin D metabolizing machinery. Pre-treatment procedures includes washing, lysis, immunocapture, cytospin, fixation, separation, spin down, filtration, dilution, distillation, concentration, inactivation of interfering compounds, centrifugation, heating, fixation, addition of reagents, or chemical treatment.

In a preferred embodiment, the semen sample is an ejaculate.

In one embodiment, the ejaculate may be lysed, fixed or otherwise modified upon direct ejaculation in to a container containing chemicals enabling this.

In an embodiment, the sample may be up-concentrated by centrifugation using a gradient such as Percoll gradient centrifugation. In another preferred embodiment, the sample may be up concentrated by centrifugation without a gradient.

In a preferred embodiment said pre-treatment procedures comprises mixing the semen sample with a phosphate buffered saline solution and subsequently spinning down the sample.

In a particular preferred embodiment the freshly delivered semen sample is centrifuged and cellular sediments collected before determining the expression level of the biomarkers. The cellular sediment may be fixed using an appropriate fixative such as but not limited to ethanol or formaldehyde.

Sperm may be "washed" by density gradient centrifugation or by a "direct swim-up" technique that doesn't involve centrifugation.

In another embodiment the sperm are separated from the seminal fluid before determining the expression level of the at least one protein of the vitamin metabolizing machinery.

In a particular preferred embodiment, no pre-treatment of the sample is necessary. In another particular preferred embodiment, the sample is a raw unmodified semen sample. The raw unmodified semen sample may be fresh. As outlined above, since the method of the invention is suited for home use, it is preferred that no pre-treatment is required.

One aspect of the present invention relates to a method wherein the semen sample may be stored for several days before determination of the expression level(s). In a preferred embodiment pre-treatment of the samples such as but not limited to cytospin of the sample prolongs the time the sample can be stored.

In a preferred embodiment, the samples may be stored for at least one day, such as at least 7 days such as at least 30 days. In another preferred embodiment, the samples may be stored for several years before detecting the expression level(s) such as at least one year, such as at least two years such as at least five years such as at least 10 years.

In one embodiment, chemicals facilitating storage are added to the semen sample.

Thus, one embodiment of the present invention enables the male individual to make the semen sample in the privacy of his own home and subsequently sending the sample to laboratory for determining the fertility potential.

### Expression level

The "expression level" or "level" as used herein refers to the absolute or relative amount of protein in a given sample. Thus, the expression level refers to the amount of protein in a sample. The expression level is usually detected using conventional detection methods.

In another preferred embodiment, the expression level refers to the total protein level of the protein in question in a semen sample.

Antibodies of the invention include polyclonal, monospecific polyclonal, monoclonal, recombinant, chimeric, humanized, fully human, single chain and/or bispecific antibodies. Antibody fragments include those portions of an anti-RANKL antibody which bind to an epitope on an RANKL polypeptide. Examples of such fragments include Fab F(ab'), F(ab)', Fv, and sFv fragments. The antibodies may be generated by enzymatic cleavage of full-length antibodies or by recombinant DNA techniques, such as expression of recombinant plasmids containing nucleic acid sequences encoding antibody variable regions.

Polyclonal antibodies are heterogeneous populations of antibody molecules derived from the sera of animals immunized with an antigen. An antigen is a molecule or a portion of a molecule capable of being bound by an antibody, which is additionally capable of inducing an animal to produce antibody capable of binding to an epitope of that antigen. An antigen can have one or more epitopes. The specific reaction referred to above is meant to indicate that the antigen will react, in a highly selective manner, with its corresponding antibody and not with the multitude of other antibodies, which can be evoked by other antigens. Polyclonal antibodies directed toward a RANKL polypeptide generally are raised in animals (e.g., rabbits or mice) by multiple subcutaneous or intraperitoneal injections of RANKL and an adjuvant.

Monoclonal antibodies (mAbs) contain a substantially homogeneous population of antibodies specific to antigens, which population contains substantially similar epitope binding sites. Such antibodies may be of any immunoglobulin class including IgG, IgM, IgE, IgA, IgD and any subclass thereof. A hybridoma producing a monoclonal antibody of the present invention may be cultivated *in vitro, in situ,* or *in vivo.* Production of high titers *in vivo* or *in situ* is a preferred method of production.

Monoclonal antibodies directed toward OPGbp/RANKL are produced using any method, which provides for the production of antibody molecules by continuous cell lines in culture. Examples of suitable methods for preparing monoclonal antibodies include hybridoma methods of Kohler et al., Nature 256, 495-497 (1975), and the human B-cell hybridoma method, Kozbor, J. Immunol. 133, 3001 (1984); Brodeur et al., Monoclonal Antibody Production Techniques and Applications, pp. 51-63 (Marcel Dekker, Inc., New York, 1987); and Harlow and Lane, Antibodies: A Laboratory Manual, Cold Spring Harbor Laboratory (1988).

A particularly preferred method for producing monoclonal antibodies directed towards OPGbp/RANKL involves immunizing the XenoMouse as described in Green, LL, J. Immunol. Methods (1999), Vol. 231, 11-25, with an OPGbp/RANKL peptide, such as a full-length human RANKL protein.

Preferred anti-RANKL or anti-RANK antibodies include monoclonal antibodies, which will inhibit partially or completely the binding of human RANKL to its cognate receptor, RANK, or an antibody having substantially the same specific binding characteristics, as well as fragments and regions thereof. Preferred methods for determining monoclonal antibody specificity and affinity by competitive inhibition can be found in Harlow et al., Antibodies: A Laboratory Manual, Cold Spring Harbor Laboratory Press, Cold Spring Harbor, N.Y., 1988), Colligan et al., eds., Current Protocols in Immunology, Greene Publishing Assoc and Wiley Interscience, N.Y., (1992, 1993), and Muller, Meth. Enzymol., 92:589-601 (1983).

Chimeric antibodies are molecules in which different portions are derived from different animal species, such as those having a variable region derived from a murine monoclonal antibody and a human immunoglobulin constant region.

The term "chimeric antibody", as used herein, includes monovalent, divalent or polyvalent immunoglobulins. A monovalent chimeric antibody is a dimer (HL) formed by a chimeric H chain associated through disulfide bridges with a chimeric L chain. A divalent chimeric antibody is tetramer (H2L2) formed by two HL dimers associated through at least one disulfide bridge. A polyvalent chimeric antibody can also be produced, for example, by employing a CH region that aggregates (e.g., from an IgM H chain, or [micro] chain).

Murine and chimeric antibodies, fragments and regions described herein may comprise individual heavy (H) and/or light (L) immunoglobulin chains. A chimeric H chain comprises an antigen binding region derived from the H chain of a non-human antibody specific for RANKL, which is linked to at least a portion of a human H chain C region (CR), such as CH1 or CH2.

A chimeric L chain according to the present invention comprises an antigen binding region derived from the L chain of a non-human antibody specific for RANKL, linked to at least a portion of a human L chain C region (CL).

Selective binding agents, such as antibodies, fragments, or derivatives, having chimeric H chains and L chains of the same or different variable region binding specificity, can also be prepared by appropriate association of the individual polypeptide chains, according to known method steps, e.g., according to Ausubel et al., eds. Current Protocols in Molecular Biology, Wiley Interscience, N.Y. (1993), and Harlow et al., Antibodies: A Laboratory Manual, Cold Spring Harbor Laboratory Press, Cold Spring Harbor, N.Y. (1988). With this approach, hosts expressing chimeric H chains (or their derivatives) are separately cultured from hosts expressing chimeric L chains (or their derivatives), and the immunoglobulin chains are separately recovered and then associated. Alternatively, the hosts can be co-cultured and the chains allowed to associate spontaneously in the culture medium, followed by recovery of the assembled immunoglobulin, fragment or derivative.

### Reference level

In the context of the present invention, the term "reference level" relates to a standard in relation to a quantity, which other values or characteristics can be compared to.

In one embodiment of the present invention, it is possible to determine a reference level by investigating the seminal fluid RANKL levels in samples from healthy subjects (in the present context fertile male subjects). By applying different statistical means, such as multivariate analysis, one or more reference levels can be calculated.

Based on these results, a cut-off may be obtained that shows the relationship between the level(s) detected and patients at risk. The cut-off can thereby be used e.g. to determine the seminal fluid RANKL levels, which corresponds to for instance an increased risk of being infertile.

### Risk Assessment

The present inventors have successfully developed a new method to predict the risk of a male subject to be infertile. To determine whether a patient has an increased risk of being infertile a cut-off (reference level) must be established. This cut-off may be established by the laboratory, the physician or on a case-by-case basis for each patient.

The cut-off level could be established using a number of methods, including: multivariate statistical tests (such as partial least squares discriminant analysis (PLS-DA), random forest, support vector machine, etc.), percentiles, mean plus or minus standard deviation(s); median value; fold changes.

The multivariate discriminant analysis and other risk assessments can be performed on the free or commercially available computer statistical packages (SAS, SPSS, Matlab, R, etc.) or other statistical software packages or screening software known to those skilled in the art.

As obvious to one skilled in the art, in any of the embodiments discussed above, changing the risk cut-off level could change the results of the discriminant analysis for each subject.

Statistics enables evaluation of the significance of each level. Commonly used statistical tests applied to a data set include t-test, f-test, or even more advanced tests and methods of comparing data. Using such a test or method enables the determination of whether two or more samples are significantly different or not.

The significance may be determined by the standard statistical methodology known by the person skilled in the art.

The chosen reference level may be changed depending on the mammal/subject for which the test is applied.

Preferably, the subject according to the invention is a human subject, such as a male subject considered at risk of being infertile.

The chosen reference level may be changed if desired to give a different specificity or sensitivity as known in the art. Sensitivity and specificity are widely used statistics to describe and quantify how good and reliable a biomarker or a diagnostic test is. Sensitivity evaluates how good a biomarker or a diagnostic test is at detecting a disease, while specificity estimates how likely an individual (i.e. control, patient without disease) can be correctly identified as not at risk. Several terms are used along with the description of sensitivity and specificity; true positives (TP), true negatives (TN), false negatives (FN) and false positives (FP). If a disease is proven to be present in a sick patient, the result of the diagnostic test is considered to be TP. If a disease is not present in an individual (i.e. control, patient without disease), and the diagnostic test confirms the absence of disease, the test result is TN. If the diagnostic test indicates the presence of disease in an individual with no such disease, the test result is FP. Finally, if the diagnostic test indicates no presence of disease in a patient with disease, the test result is FN.

### Sensitivity

As used herein the sensitivity refers to the measures of the proportion of actual positives which are correctly identified as such.

Usually the sensitivity of a test can be described as the proportion of true positives of the total number with the target disorder i.e. a fertility potential below normal. All patients with the target disorder are the sum of (detected) true positives (TP) and (undetected) false negatives (FN).

### Specificity

As used herein the specificity refers to measures of the proportion of negatives which are correctly identified. The ideal diagnostic test is a test that has 100% specificity and therefore no false positive results, and 100% sensitivity, and i.e. detects all mammals below normal and therefore no false negative results.

For any test, there is usually a trade-off between each measure. For example in a manufacturing setting in which one is testing for faults, one may be willing to risk discarding functioning components (low specificity), in order to increase the chance of identifying nearly all faulty components (high sensitivity). This trade-off can be represented graphically using a ROC curve.

Selecting a sensitivity and specificity it is possible to obtain the optimal outcome in a detection method. In determining the discriminating value distinguishing mammals having a fertility potential below normal, the person skilled in the art has to predetermine the level of specificity. The ideal diagnostic test is a test that has 100% specificity, i.e. only detects mammals with a fertility potential below normal and therefore no false positive results, and 100% sensitivity, and i.e. detects all mammals with a fertility potential below normal and therefore no false negative results. However, due to biological diversity no method can be expected to have 100% sensitive without including a substantial number of false negative results.

The chosen specificity determines the percentage of false positive cases that can be accepted in a given study/population and by a given institution. By decreasing specificity an increase in sensitivity is achieved. One example is a specificity of 95% that will result in a 5% rate of false positive cases. With a given prevalence of 1% of e.g. a fertility potential below normal in a screening population, a 95% specificity means that 5 individuals will undergo further physical examination in order to detect one (1) fertility potential below normal if the sensitivity of the test is 100%.

As will be generally understood by those skilled in the art, methods for screening for fertility potentials are processes of decision making and therefore the chosen specificity and sensitivity depends on what is considered to be the optimal outcome by a given institution/clinical personnel.

### Method for determining a likely effect of a treatment to improve male fertility

As also outlined above, the present invention provides novel biomarkers which can be used for determining a likely effect of a treatment to improve male fertility. Thus, an aspect of the invention relates to a method for determining a likely effect of a treatment to improve male fertility, the method comprising
- determining the level of AMH and/or inhibin B in a biological sample from a male subject;
- comparing said determined AMH level and/or inhibin B level to a corresponding reference level; and
- determining that said subject will
   ∘ likely benefit from treatment with an antagonist or inhibitor of RANKL, if said level is equal to or higher than said corresponding reference level; or
   ∘ likely not benefit from treatment with an antagonist or inhibitor of RANKL, if said level is lower than said corresponding reference level.

In a preferred embodiment said reference level is
- above 30 pmol/l AMH in blood serum, such as at least 35 pmol/l AMH or such as at least 40 pmol/l AMH, or such as at least 50 pmol/l AMH; and/or
- above 5 pmol/l in seminal fluid.

In another preferred embodiment, at least the level of AMH is determined.

In yet another embodiment, at least the level of inhibin B is determined.

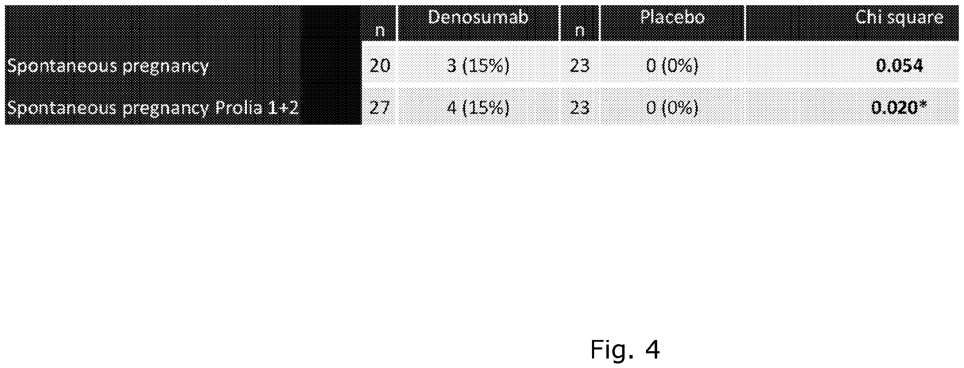

As can be seen in example 1 and figure 5, when a male subject has an AMH level e.g. above 30 or above 40 pmol/l AMH in serum, the effect of treatment with an RANKL antagonist or inhibitor is much higher (in relation to improving male fertility parameters) than when the male subject has a lower AMH level. This is also the case for spontaneous pregnancies (the ultimate marker of male fertility), which is higher in infertile men with serum AMH > 30 pmol/treated with a RANKL inhibitor compared with placebo (Figure 4).

AMH levels can be determined by different means. Thus, in an embodiment, said determination of the level of AMH is performed using a method selected from the group consisting of immunohistochemistry, immunocytochemistry, FACS, ImageStream, Western Blotting, qPCR, RT-PCR, qRT-PCR, ELISA, Luminex, Multiplex, Immunoblotting, TRF-assays, immunochromatographic lateral flow assays, Enzyme Multiplied Immunoassay Techniques, RAST test, Radioimmunoassays, immunofluorescence, LC-MS/MS and immunological dry stick assays, preferably lateral flow assays

In a preferred embodiment, said determination is performed by immunocytochemistry. In another preferred embodiment, said determination is performed by ELISA, LC-MS/Ms or lateral flow test.

Different reference levels may be selected by a clinician dependent on a desired specificity and sensitivity. Thus, in an embodiment,
- said reference level in blood serum is in the range 30 to 100 pmol/l AMH, such as in the range 30 to 90 pmol/l AMH, such as in the range 30 to 80 pmol/l AMH, such as in the range 35 to 80 pmol/l AMH, such as in the range 40 to 80 pmol/l AMH, or such as in the range 50 to 80 pmol/l AMH; and/or
- said reference level in seminal fluid is in the range in the range 5-5000 pmol/l, such as 5-1000 pmol/l, such 5 to 600 pmol/l AMH, such as in the range 5 to 100 pmol/l AMH (data not shown).

Different types of antagonists or inhibitors of RANKL exist. Thus, in another embodiment, the antagonist or inhibitor of RANKL is a molecule having binding affinity for RANKL, such as wherein the RANKL binding moiety binds to soluble RANKL, such as binds to full length RANKL, or truncated versions of RANKL, such as binds to C-terminal or N-terminal truncated versions of RANKL, preferably binds to the C-terminal of RANKL.

In yet an embodiment, the antagonist or inhibitor of RANKL having binding affinity for RANKL, binds RANKL at the same epitope as Denosumab or OPG.

In yet a further embodiment, the antagonist or inhibitor of RANKL having binding affinity for RANKL, binds RANKL with a binding affinity similar to Denosumab or OPG or with a binding affinity higher than Denosumab or OPG.

In yet a further embodiment, the antagonist or inhibitor of RANKL is selected from the group consisting of polyclonal antibody, a monoclonal antibody, an antibody wherein the heavy chain and the light chain are connected by a flexible linker, an Fv molecule, an antigen binding fragment, a Fab fragment, a Fab' fragment, a F(ab')2 molecule, a fully human antibody, a humanized antibody, a chimeric antibody and a single-domain antibody (sdAb) (nanobody), and small molecule inhibiting RANKL function.

In yet another embodiment, said inhibitor of RANKL is selected from the group consisting of Denosumab or fragments thereof (able to bind to RANKL), OPG or fragments thereof (able to bind to RANKL). In the example data provided the RANKL inhibitor Denosumab has been tested. Denosumab has previously been shown to be efficient in the treatment of male infertility (WO 2015/018421).

The inclusion of other biomarkers in the assessment may improve the predictive value of the assay. Thus, in an embodiment, the method further comprises
- determining the level of Inhibin B, and/or RANKL and/or OPG and/or RANKL/OPG ratio (or vice versa) and/or AMH/inhibin B ratio (or vice versa) and/or AMH/testosterone ratio (or vice versa) in a biological sample from the male subject;
- comparing said determined level of Inhibin B, and/or RANKL and/or OPG and/or RANKL/OPG ratio AMH/inhibin B ratio (or vice versa) and/or AMH/testosterone ratio (or vice versa) to a corresponding reference level; and
- determining that said subject will
   ∘ likely benefit from treatment with an antagonist or inhibitor of RANKL, if said one or more levels are equal to or higher than said one or more reference levels; or
   ∘ likely not benefit from treatment with an antagonist or inhibitor of RANKL, if said on or more levels are lower than said reference level.

As shown in example 1, Inhibin B, and/or RANKL and/or OPG and/or RANKL/OPG (or vice versa) and/or AMH/inhibin B ratio (or vice versa) ratio can all improve the predictive value of the assay, albeit AMH appears to have a stronger predictive when used alone compared to the other listed biomarkers used alone. The skilled person would know how to incorporate such multivariate analysis in an assay. Further. EP3244911 B1 discloses a method for determining a likely effect of a treatment to improve male fertility, the method comprises determining the level of OPG in a blood serum sample.

In a related embodiment, said further one or more levels are determined in the same sample or sample type as the level of AMH is determined.

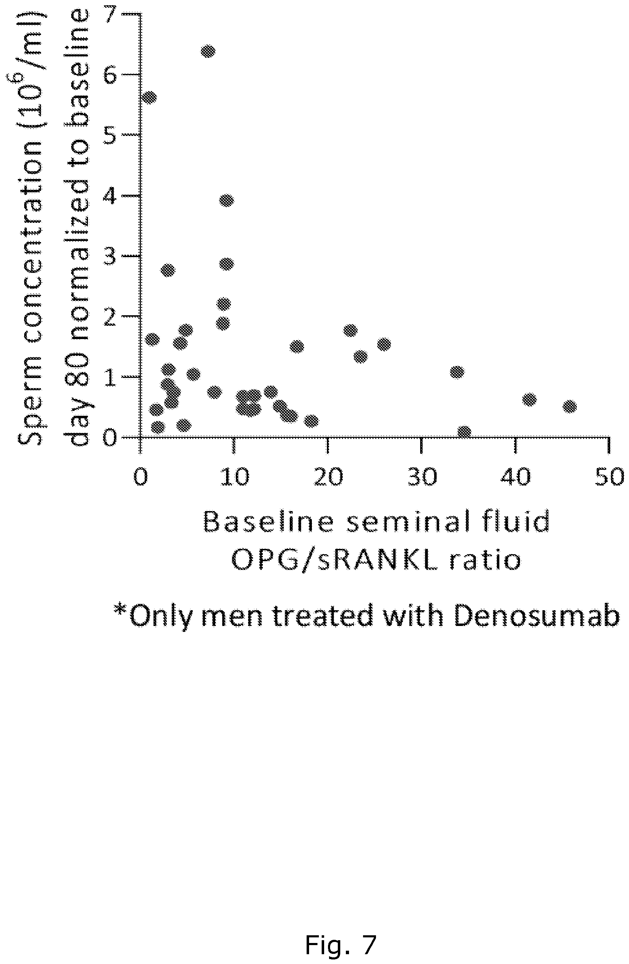

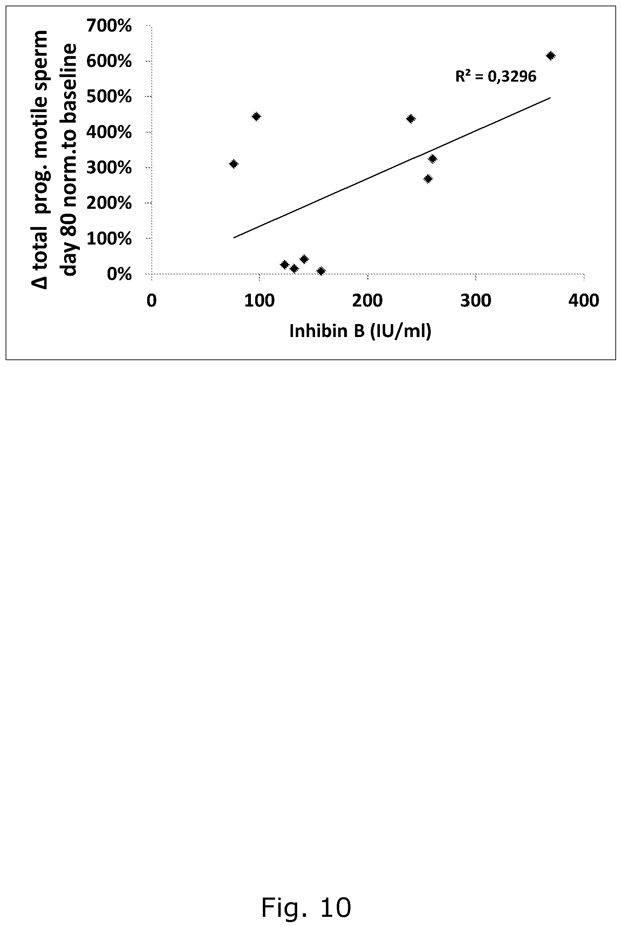

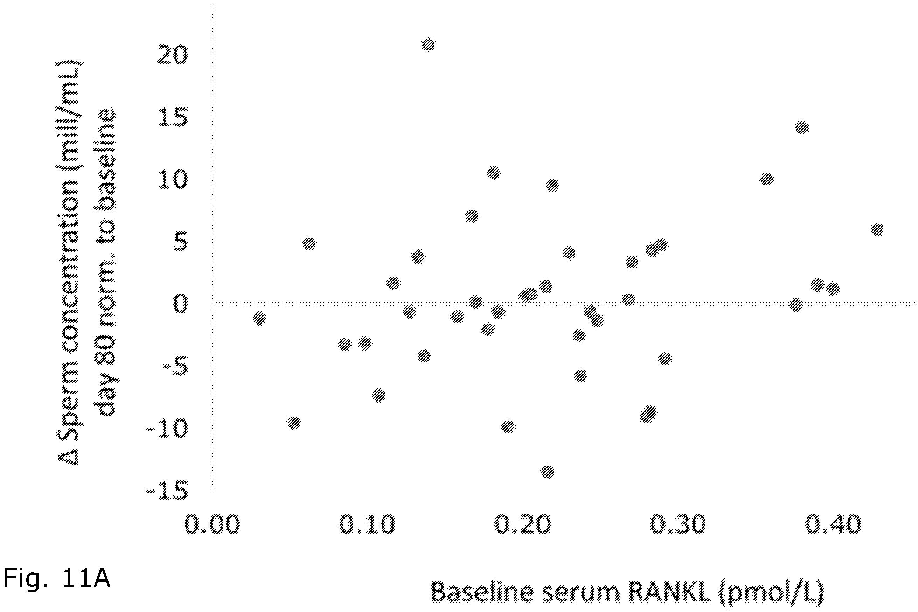

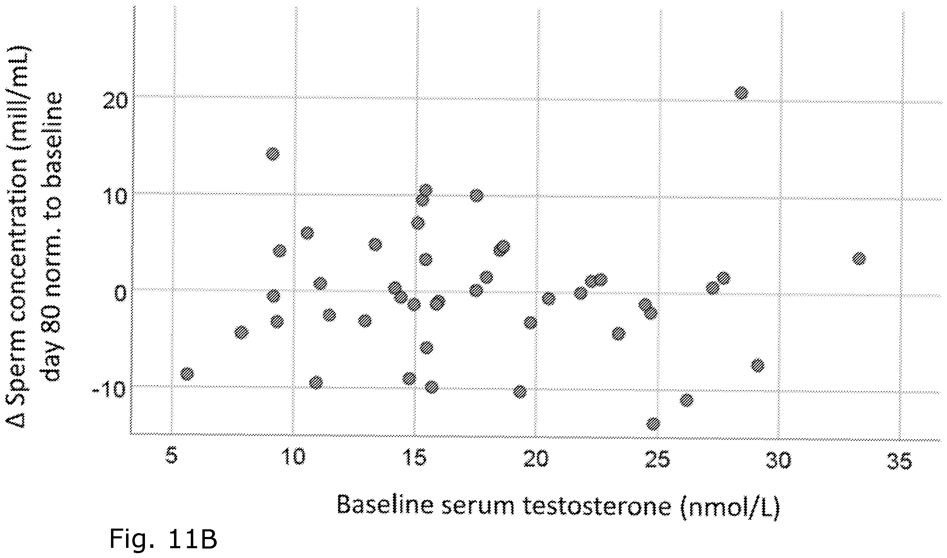

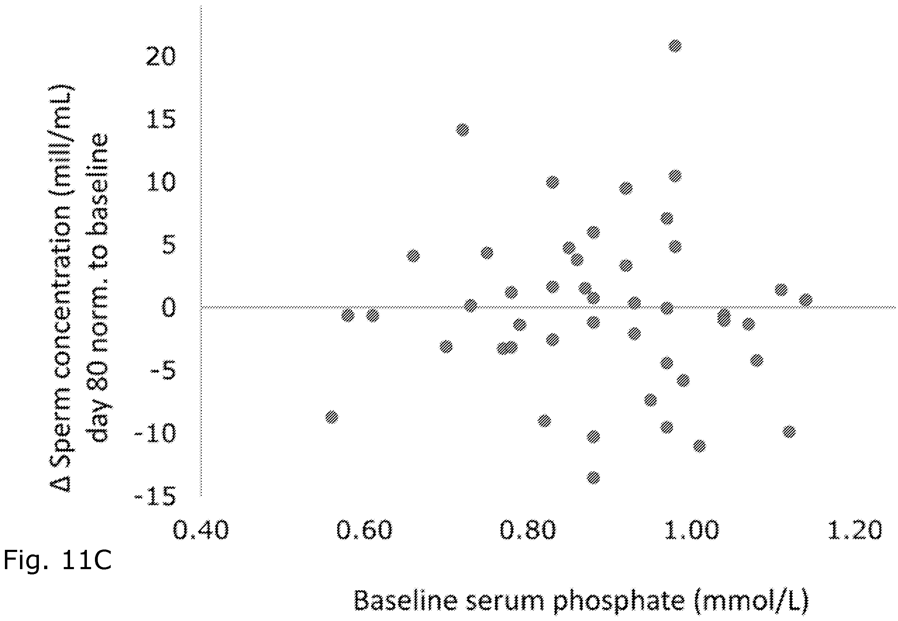

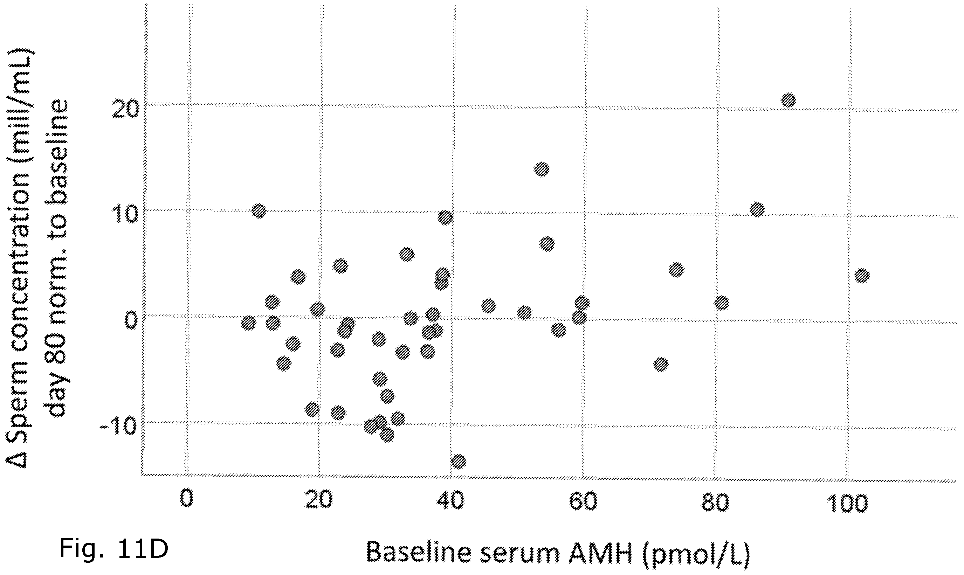

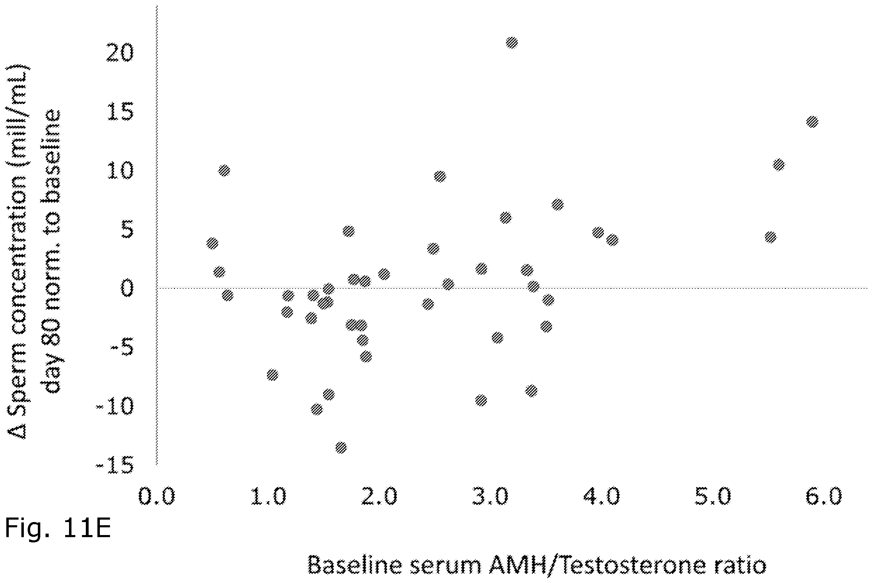

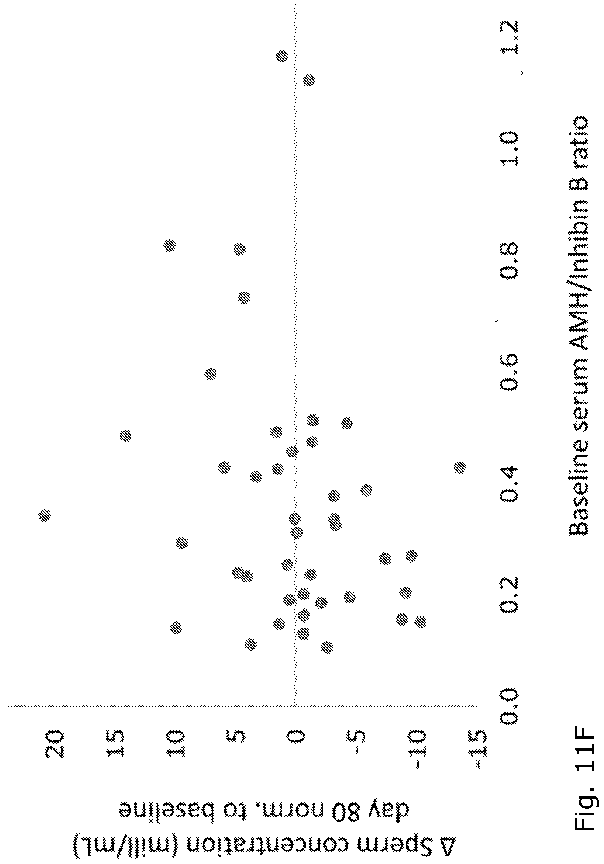

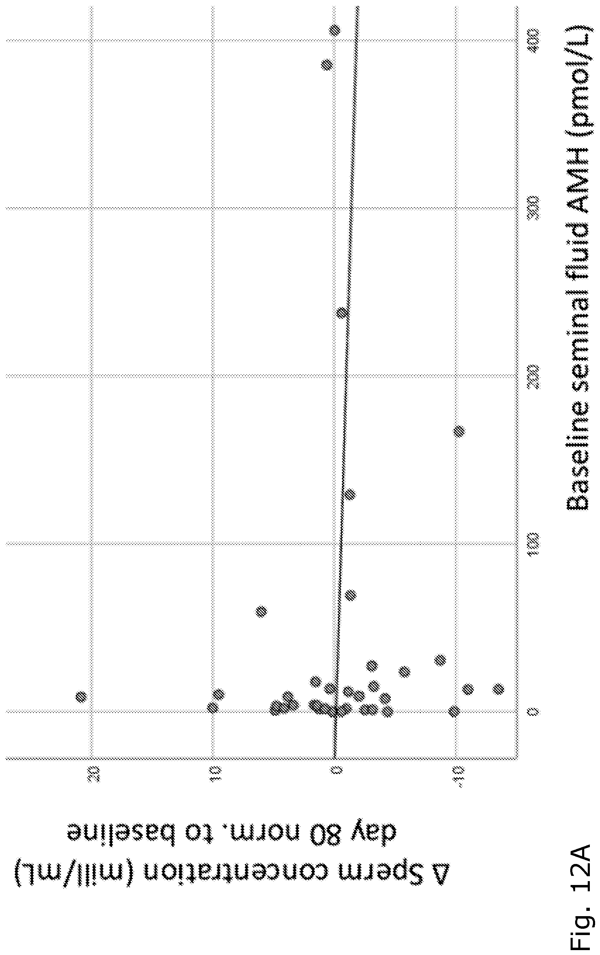

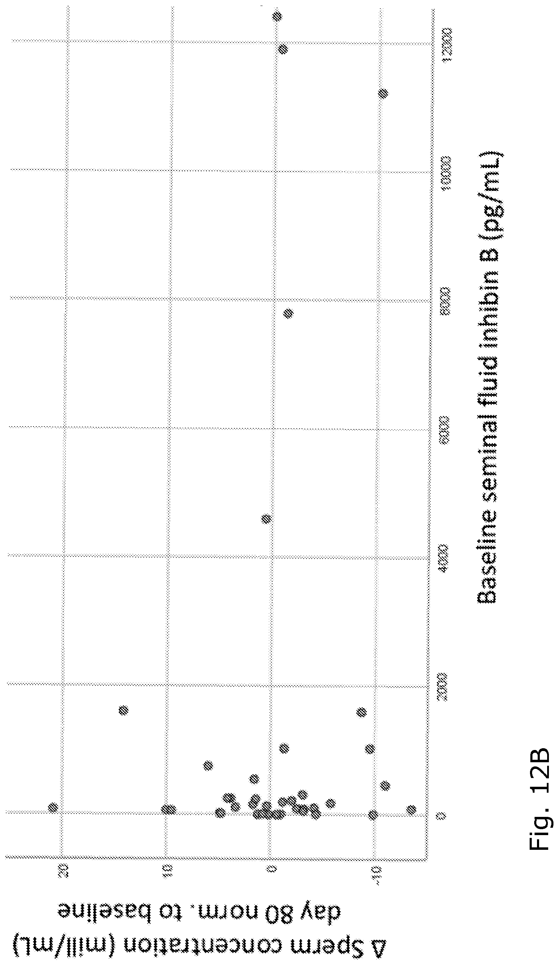

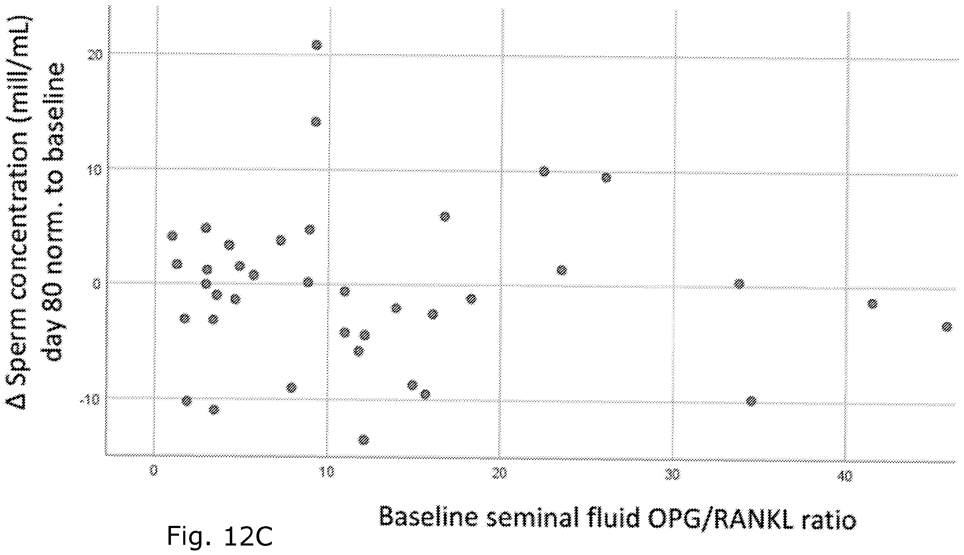

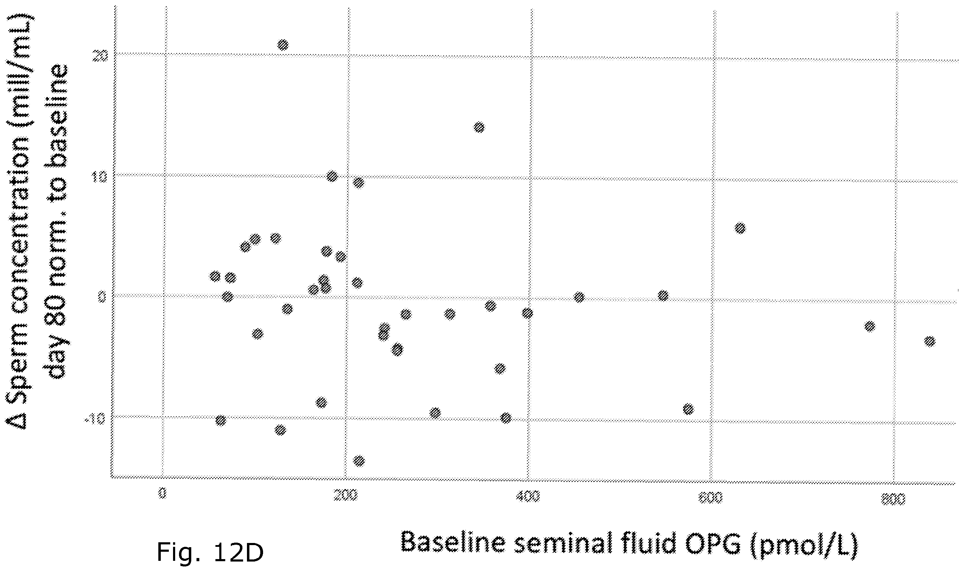

Relevant reference levels for the other biomarkers are:
- said reference level in blood serum is at least 50 IU/ml Inhibin B, such as at least 100 IU/ml Inhibin B or such as at least 200 IU/ml Inhibin B, or such as in the range 100 to 500 IU/ml Inhibin B, or such as in the range 500 to 500 IU/ml Inhibin B(figure 10 shows data for inhibin B); and/or
- said reference level in seminal fluid is at least 20 IU/ml Inhibin B, such as at least 100 IU/ml Inhibin B or such as at least 200 IU/ml Inhibin B, or such as in the range 20 to 500 IU/ml Inhibin B, or such as in the range 100 to 1000 IU/ml Inhibin B; and/or (see figure 12).
   and/or
- said reference level in blood serum is in the range 2 to 5 pmol/l OPG, such as in the range 3-5 pmol/l OPG such as in the range 3-4 pmol/l OPG (EP3244911 B1 discloses data for OPG); and/or
- said reference level in seminal fluid is in the range in the range 0 to 220 pmol/l OPG, or such as in the range 10 to 400 pmol/l OPG or such as in the range 10 to 700 pmol/l OPG (figure 12D)
   and/or
- said reference level in blood serum is in the range 0.1 to 0.5 pmol/l soluble RANKL (sRANKL), such as in the range 0.2-0.4 pmol/l RANKL (see figure 11); and/or
- said reference level in seminal fluid is in the range in the range 5 to 50 pmol/l sRANKL, such as in the range 10 to 40 pmol/l RANKL. (figure 7 shows data for OPG/RANKL ratio)
   and/or
- said reference level in blood serum is an OPG/RANKL below 30, such as below 20, or such as below 15; and/or
- said reference level in blood seminal fluid is an OPG/RANKL below 30, such as below 20, or such as below 15 (See figure 12).

In a preferred embodiment, RANKL levels are levels of sRANKL. In a related embodiment, the reference level is in the range 1-76 pmol/L seminal sRANKL, such as 20-76 pmol/l seminal sRANKL, 40-76 pmol/l seminal sRANKL, 1-50 pmol/l seminal sRANKL, 1-30 pmol/l seminal sRANKL, or 1-20 pmol/l seminal sRANKL.

In a further embodiment, levels of serum testosterone and/or serum phosphate may also be included in the method.

Thus, in a more specific embodiment, the method further comprises
- determining the level of testosterone and/or phoshate in a biological sample from the male subject;
- comparing said determined level of testosterone and/or phoshate to a corresponding reference level; and
- determining that said subject will
   ∘ likely benefit from treatment with an antagonist or inhibitor of RANKL, if said one or more levels are equal to or higher than said reference level; or
   ∘ likely not benefit from treatment with an antagonist or inhibitor of RANKL, if said on or more levels are lower than said reference level.

In another embodiment, the reference level for serum testosterone is > 10 nmol/l, such as in the range 10-40 nmol/l. Thus, In an embodiment, serum testosterone > 10 nmol/l will select the men who will benefit from the treatment, such as in the range 10-40 nmol/l (see figure 11). Thus, the combination of AMH and testosterone could be a strong predictive combination for a likely benefit from treatment with an antagonist or inhibitor of RANKL.

In yet another embodiment, the reference level for serum phosphate is > 0.6 mmol/l, such as in the range 0.6-1.0 mmol/l. Thus, in an embodiment, serum phosphate > 0.6 mmol/l will select the men who will benefit from the treatment or such as in the range 0.6-1.0 mmol/l (see figure 11). Thus, the combination of AMH and phosphate could be a strong predictive combination for a likely benefit from treatment with an antagonist or inhibitor of RANKL.

It is of course to be understood that AMH can be combined with the other predictive markers indicated in here.

It is of course also to be understood that Inhibin B can be combined with the other predictive markers indicated in here.

The different sample types may be processed before use. Thus, in an embodiment, the semen sample is an unmodified, diluted, washed or purified semen sample, preferably an unmodified semen sample.

Male infertility may have different causes. Thus, in a preferred embodiment, said subject suffers from oligospermia.

In the case a subject is considered treatable after the assessment according to the invention, a treatment may be initiated. Thus, in an embodiment not claimed the method further comprises administering to said subject in need of treatment, an antagonist or inhibitor of RANKL, if said AMH level is equal to or higher than said reference level.

### Uses

In another aspect, the invention relates to the use of AMH and/or inhibin B as a biomarker for determining a likely effect of a treatment to improve male fertility.

In an embodiment, treatment is with an antagonist or inhibitor of RANKL.

In yet an embodiment, a level above 30 pmol/l AMH in blood serum is indicative of a likely positive effect on male infertility of the treatment with an antagonist or inhibitor of RANKL.

In a further embodiment, a level in blood serum of at least 50 IU/ml Inhibin B, such as at least 100 IU/ml Inhibin B or such as at least 200 IU/ml Inhibin B, or such as in the range 100 to 500 IU/ml Inhibin B, or such as in the range 50 to 500 IU/ml Inhibin B is indicative of a likely positive effect on male infertility of the treatment with an antagonist or inhibitor of RANKL (figure 10 shows data for inhibin B);
and/or
a level in seminal fluid is at least 20 IU/ml Inhibin B, such as at least 100 IU/ml Inhibin B or such as at least 200 IU/ml Inhibin B, or such as in the range 20 to 1000 IU/ml Inhibin B, or such as in the range 100 to 5000 IU/ml Inhibin B is indicative of a likely positive effect on male infertility of the treatment with an antagonist or inhibitor of RANKL (figure 12 shows data for inhibin B).

It is of course to be understood that AMH and/or Inhibin B can be combined with the other predictive markers mentioned in here. Thus, embodiments of other aspects of this invention can be combined with this aspect.

### Treatment of subgroup of infertile men

As outline above and in the example section, in the present invention a specific subgroup of infertile men has been identified who are more susceptible to a treatment against infertility using an antagonist or inhibitor of RANKL as the medicament.

Thus, a further aspect of the invention relates to a composition comprising an antagonist or inhibitor of RANKL for use in the treatment of male infertility in subject, wherein said subject has:
- above 30 pmol/l in blood serum of AMH, such as at least 35 pmol/l AMH or such as at least 40 pmol/l AMH; and/or
- above 5 pmol/l AMH in seminal fluid;
   and/or
- a level in blood serum of at least 50 IU/ml Inhibin B, such as at least 100 IU/ml Inhibin B or such as at least 200 IU/ml Inhibin B, or such as in the range 100 to 500 IU/ml Inhibin B, or such as in the range 50 to 500 IU/ml Inhibin B; and/or
- a level in seminal fluid is at least 20 IU/ml Inhibin B, such as at least 100 IU/ml Inhibin B or such as at least 200 IU/ml Inhibin B, or such as in the range 20 to 1000 IU/ml Inhibin B, or such as in the range 100 to 5000 IU/ml Inhibin B.

As outlined above, different types of antagonists or inhibitors of RANKL exist. Thus, in another embodiment, the antagonist or inhibitor of RANKL is a molecule having binding affinity for RANKL, such as wherein the RANKL binding moiety binds to soluble RANKL, such as binds to full length RANKL, or truncated versions of RANKL, such as binds to C-terminal or N-terminal truncated versions of RANKL, preferably binds to the C-terminal of RANKL.

In yet an embodiment, the antagonist or inhibitor of RANKL having binding affinity for RANKL, binds RANKL at the same epitope as Denosumab or OPG.

In yet a further embodiment, the antagonist or inhibitor of RANKL having binding affinity for RANKL, binds RANKL with a binding affinity similar to Denosumab or OPG or with a binding affinity higher than Denosumab or OPG.

In yet a further embodiment, the antagonist or inhibitor of RANKL is Denosumab or OPG or active fragments or variants thereof, which binds to RANKL. In an embodiment, the antagonist or inhibitor of RANKL is a biosimilar.

In the present context, a biosimilar is to be understood as a product highly similar to Denosumab in binding function. Preferably, the biosimilar is a RANKL binding molecule (such as protein), which binds similar to Denosumab to RANKL. An example could be a nanobody developed against the same epitope on RANKL as Denosumab.

In yet a further embodiment, the antagonist or inhibitor of RANKL is selected from the group consisting of polyclonal antibody, a monoclonal antibody, an antibody wherein the heavy chain and the light chain are connected by a flexible linker, an Fv molecule, an antigen binding fragment, a Fab fragment, a Fab' fragment, a F(ab')2 molecule, a fully human antibody, a humanized antibody, a chimeric antibody and a single-domain antibody (sdAb) (nanobody) or a small molecule inhibiting RANKL.

It is of course to be understood that AMH and/or Inhibin B can be combined with the other predictive markers mentioned in here, to determine the subgroup of subjects for which a composition comprising an antagonist or inhibitor of RANKL for use in the treatment of male infertility in subject will be beneficial. Thus, embodiments of other aspects of this invention can be combined with this aspect

### Further aspect

In an additional aspect, described herein but not part of the invention is a method of treating male infertility in a subject in need thereof, said method comprising administering an antagonist or inhibitor of RANKL to said subject if said subject has a level of AMH of:
- above 30 pmol/l in blood serum, such as at least 35 pmol/l AMH or such as at least 40 pmol/l AMH; and/or
- above 5 pmol/l in seminal fluid.
   and/or
- at least 50 IU/ml Inhibin B in blood serum, such as at least 100 IU/ml Inhibin B or such as at least 200 IU/ml Inhibin B, or such as in the range 50 to 500 IU/ml Inhibin B, or such as in the range 200 to 500 IU/ml Inhibin B; and/or
- at least 20 IU/ml Inhibin B in seminal fluid, such as at least 100 IU/ml Inhibin B or such as at least 200 IU/ml Inhibin B, or such as in the range 20 to 500 IU/ml Inhibin B, or such as in the range 20 to 10000 IU/ml Inhibin B.

It is of course to be understood that the reference levels from the other aspect of the invention may also find use in this aspect.

It should be noted that embodiments and features described in the context of one of the aspects of the present invention also apply to the other aspects of the invention.

The invention will now be described in further details in the following non-limiting examples.

### Examples

### Example 1 - Denosumab and Male Infertility: a Randomized Controlled Double-blinded Intervention Study

### Aim of study

To determine the significance of systemic RANKL inhibition for male reproduction, conducting a clinical controlled randomized double blinded intervention study on infertile men, to investigating whether Denosumab (Prolia) can increase semen quality and to investigate what subgroup of infertile men that might benefit from treatment.

### Materials and methods

### Design:

Single center, prospective randomized double blinded* clinical controlled intervention.

### Patients and methods:

95 infertile men, otherwise healthy, will be recruited among men referred to investigation for infertility at the outpatient clinic at Dpt. of Growth and Reproduction, Rigshopitalet. Neither choosing to participate, nor declining, will affect further outpatient clinic treatment.

The 95 participants are randomly allocated to receiving either
Denosumab s.c. injection 60 mg x1, or
Placebo, Sodium Chloride s.c. injection x 1
All participants deliver semen samples, blood samples and have DXA-scan performed before and after intervention. All receive supplements with Vitamin D and calcium to avoid hypocalcaemia, a known, temporary side effect to Denosumab.
*double blinded studies require that placebo and active substance are identical. Placebo can only be obtained from Amgen and we were not able to receive or purchase placebo. Instead, the study is double blinded to patient, medical doctors and all who participates in data-handling and analyses - only one nurse giving the medicine will be unblinded. She will know whether she injects active substance or placebo.

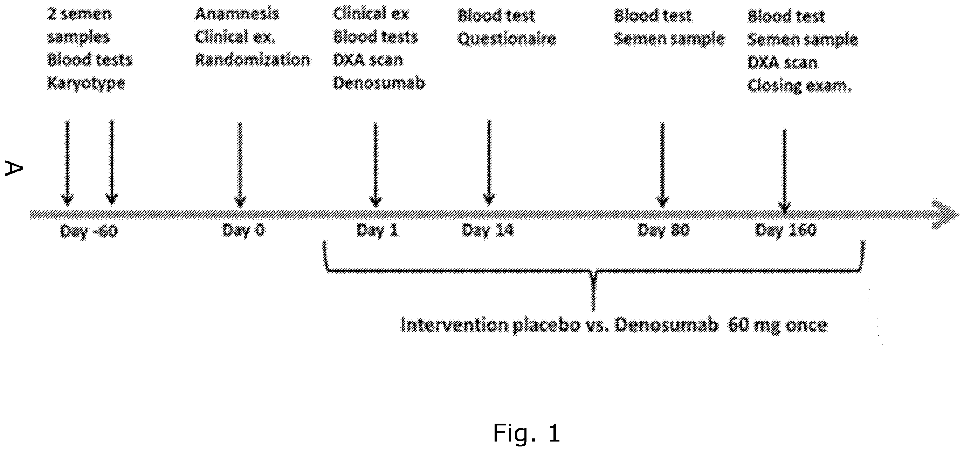

An overview of the study design is shown in figure 1.

### Inclusion criteria:

Men > 18 years of age referred due to infertility in need for further investigation with:
Sperm concentration ≥ 0,05 mio./ml.
Sperm concentration <15 mio./ml or
<40 % motile spermatozoa (ABC) or
<4 % morphologically normal sperm (strict criteria)

### Exclusion criteria:

Men with chronic diseases (diabetes mellitus, thyroid disease, endocrine diseases requiring treatment, malignant diseases or diseases known to be affected by- or interfere with vitamin D supplements (granulomatous diseases such as sarcoidosis, tuberculosis, wegeners, vasculitis as well as inflammatory bowel diseases e.g. chron's disease or ulcerative colitis etc.).

Men with active or previous malignant disease
Any case with indication for testis biopsy,
Total calcium < 2.14 mmol/l
25-OH Vitamin D < 25 nmol/L
Poor dental status or dental implants
Men with obstructive oligospermia or who has been vasectomized
Serum Inhibin B < 30 pg/ml
Abnormal karyotype
Patients practicing excessive exercise

### Sample size calculation and statistics:

With a test level of 5% (level of significance), a power of 80% and 40 men in each group will we be able to detect a change in total number of motile sperm of 75%, and a change in sperm concentration (indexed to baseline value) of 25%.

We expected to include a total of 95 men, to take into account the possibility of dropouts. This corresponds to a dropout rate of 10%, in case the rate is lower, chances of detecting a significant difference will be above 80%.

Subgroup analysis will be essential in this study because we expect that around 60% of the men will benefit from treatment with Denosumab. Analyses after stratification into subgroups will therefore be used to identify a marker for selecting the men who will benefit from the treatment Subjects will be grouped according to their serum AMH, Inhibin B, Inhibin B/AMH ratio, OPG and/or RANKL, RANKL/OPG ratio, their DNA-fragmentation-Index prior to the start of intervention. Afterwards other potential predictors of treatment response will be tested such as BMI, S-calcium, s-phosphate, BMD, semen quality, Inhibin b, FSH at day 80 and day 160. For Serum OPG such stratification could be in two groups separated by for instance 3 pg/ml or three groups, <3, 3-4 and above 4pg/ml. Likewise for RANKL and RANKL/OPG ratio and for DNA-fragmentation-Index stratification could also be in two groups separated by e.g. DFI of 40%. The subgroup analyses will be in accordance with normal clinical practice and stratification in appropriate groups according to the clinical or highest/lowest groups.

### OUTCOME MEASUREMENTS

### Endpoints:

### Primary endpoints

- Change in semen production (total motile spermatozoa, progressive motile spermatozoa, sperm count, sperm concentration)

### Secondary endpoints

Change in semen quality (-motility, -morphology, semen volume)
Change in DNA fragmentation (DFI)rate in sperm
Change in serum Inhibin-B
Change in serum levels of reproductive hormones (FSH, LH, AMH, testosterone, estradiol and SHBG)
Change in serum prolactin level
Change in fasting glucose level
Change in fastin insulin level
Change in c-peptide
Change in HbA1c
Change in lipid profile
Change in bone mineral density evaluated by DXA.
Change in serum level of inactive vitamin D, 1,25(OH)2D3, 25-OHD3, 24,25(OH)2D3, PTH, alkaline phosphatase, ionized calcium, phosphate, FGF23, Klotho, osteocalcin, matrix gla, osteopontin, calcitonin, pnp, procollagen III, OPG, RANKL, Sclerostin as well as other bone marker.
Change in choice of assisted reproductive assistance technique as well as conceived pregnancies
Change in spontaneous conception rate
Change in live birth rate
Change in number of spermatozoa expressing RANKL
Change in semen pH, HCO3-, calcium, zink, phosphate, RANKL, RANK, OPG, FGF23, Klotho, osteocalcin, osteopontin. VDB, 24,25OH2
Difference in infection rate in the two groups

### SETTING, SCIENTIFIC PLAN AND RECRUITMENT

Participants will be included among men referred to the Department of Growth and Reproduction (dept. of GR), Rigshospitalet (RH) for evaluation of male infertility
This is a prospective, double blinded, two-arm randomized controlled trial. Group of intervention: Each man will receive Denosumab 60 mg x 1 sc at day 1 only Group receiving placebo: Will follow the same chain of events, although Denosumab will be replaced with saline

All men will be offered the same vitamin D and calcium supplementation starting prior to initiation of the treatment start of Denosumab or placebo: vitamin D 15 microgram and calcium 400 mg once daily.

### PARTICIPANTS

100 men were included in the study and half will be randomized to active treatment. The investigators expect a small drop out (<5) because of high motivation and no adverse effects.

### ANALYSIS AND INTERVENTION

Reproductive hormones and semen is analyzed at dept. of GR, Rigshospitalet.

### ETHICS AND SIDE EFFECTS

All the patients will have full-filled their investigation, before they are invited to the study. They will be informed of potential adverse effects, and they can leave the trial at any point without any consequences.

Denosumab is proven to be safe in several randomized studies, and is already approved by both FDA and EMA. All side effects is closely monitored, registered and in case of either unexpected frequency of adverse effects or unexpected clinical complications it will be published.

Based on previous fertility studies at our department we expect a large proportion of the out clinic-patients will wish to participate. In our opinion, the outlined study is ethical sound, and we believe that in order to generate future recommendations, randomized clinical trials are necessary.

| | |
|---|---|
| Study Type | Interventional |
| Study Phase | Phase 1/2 |
| Study Design Allocation: | Randomized |
| Intervention Model: | Parallel Assignment |

NaCl 0.9% injection is used as placebo
   Other Name: Prolia
   Active Comparator: Denosumab
subcutaneous injection with 60 mg Denosumab once
   Intervention: Drug: Denosumab
   Placebo Comparator: Placebo
subcutaneous injection with NaCl once
   Intervention: Drug: Denosumab

### Biochemical analyses:

All blood samples were drawn fasting during morning hours (8.00 to 10.00 a.m.). To determine FSH and LH levels we used a time-resolved immuno-fluorometric assay (Delfia; Wallac, Turku, Finland) and for inhibin-B we used a specific two-sided enzyme linked immunoassay (inhibin B genII, Beckman Coulter, USA). The CVs were<6%,<4%, <4% and < 11% for SHBG, FSH, LH and Inhibin B, respectively. AMH was measured using the Beckman Coulter enzyme immunometric assay (Immunotech, Beckman Coulter, Marseilles, France) with CV<8%. Testosterone and sex hormone binding globulin (SHBG) were analysed in chemiluminescent immune assays (Access, Beckman Coulter, USA), and estradiol levels were analysed by radioimmunoassay (Coat-a-Count, Siemens and Pantex, Santa Monica, CA, respectively) with CV of 6, 8 and 13%, respectively. Measurements of OPG and soluble RANKL in both serum and seminal fluid were performed using kits from Biomedica (CV 3%, CV 5%).

### Results

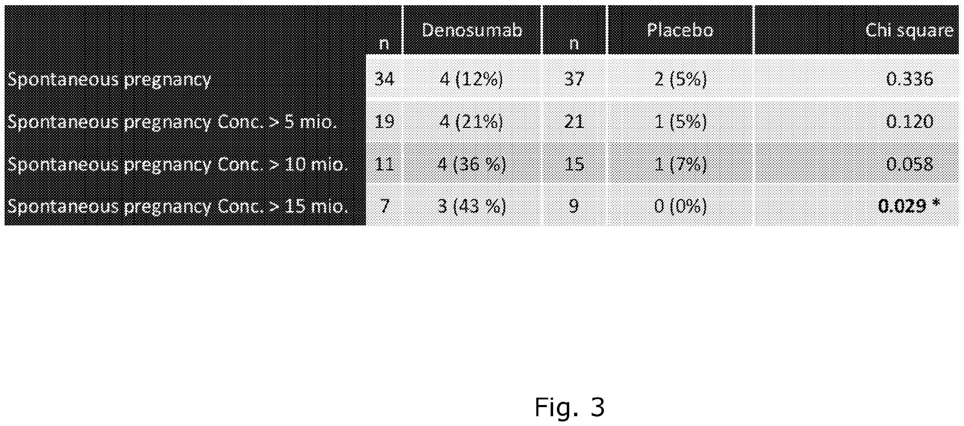

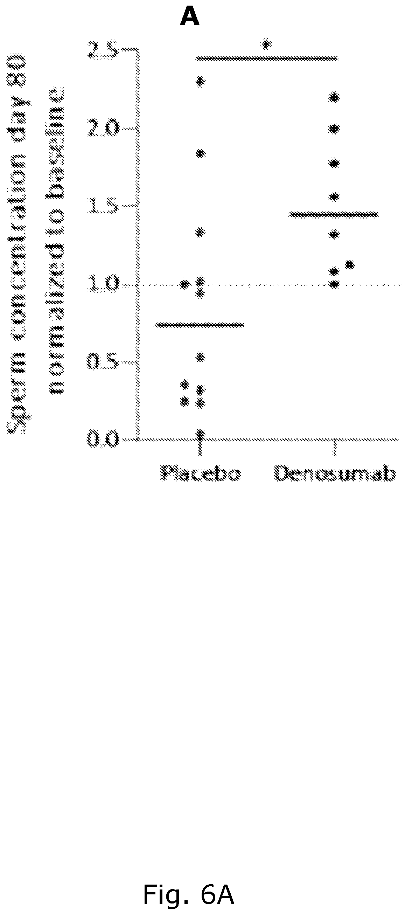

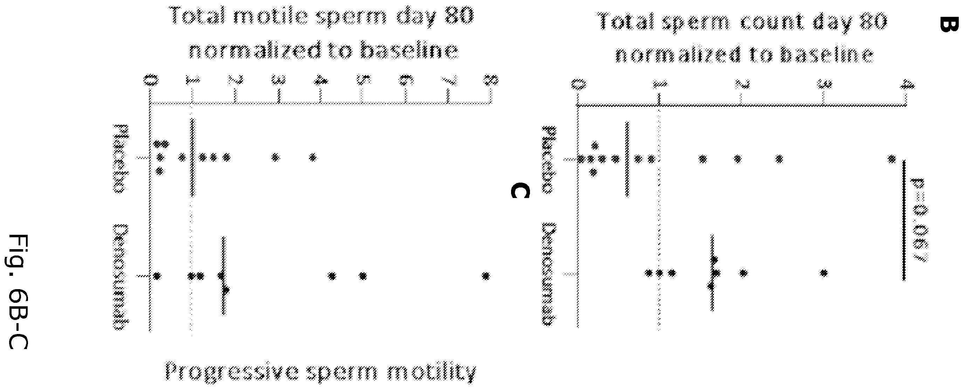

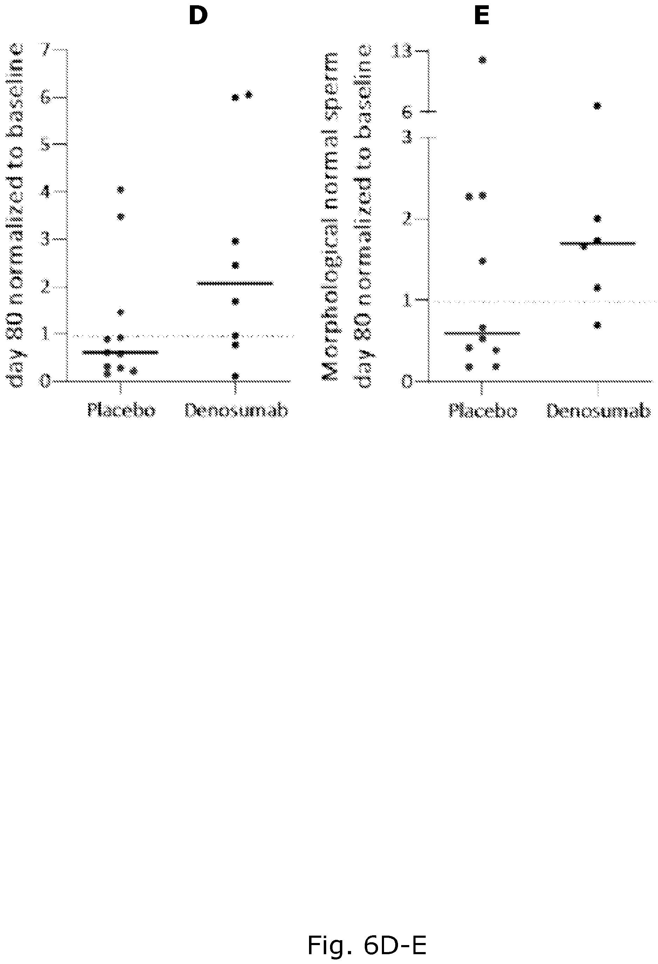

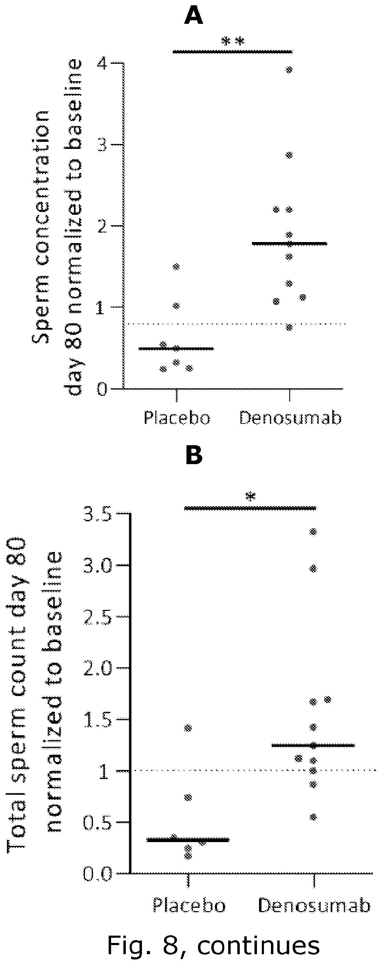

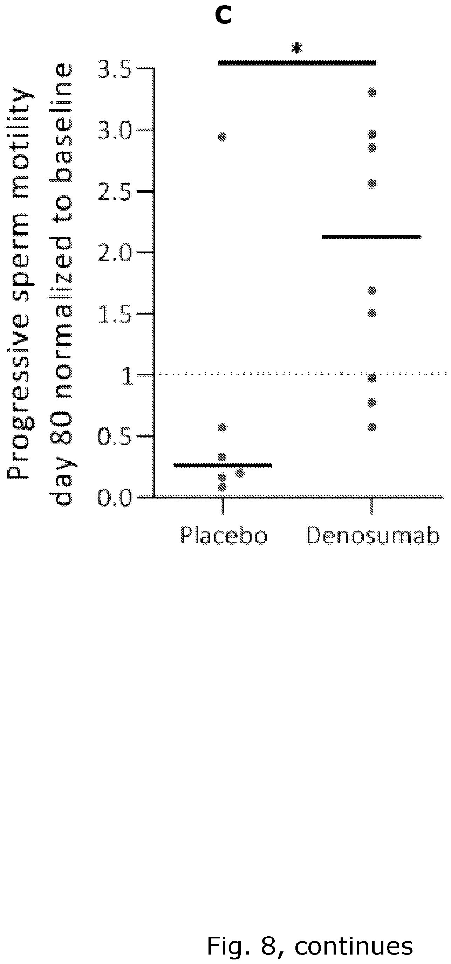

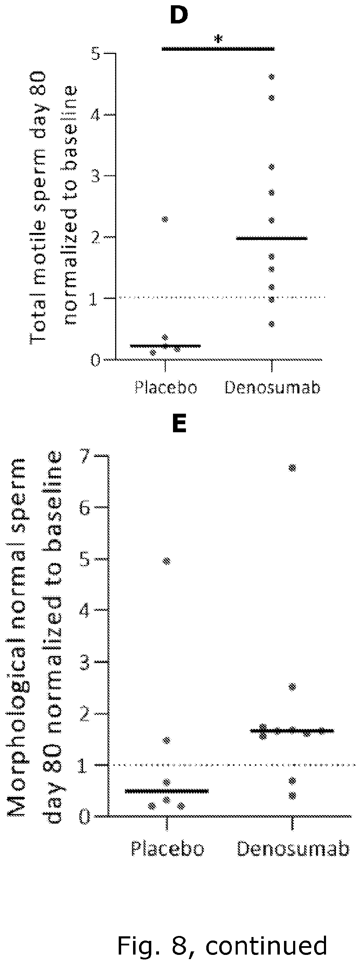

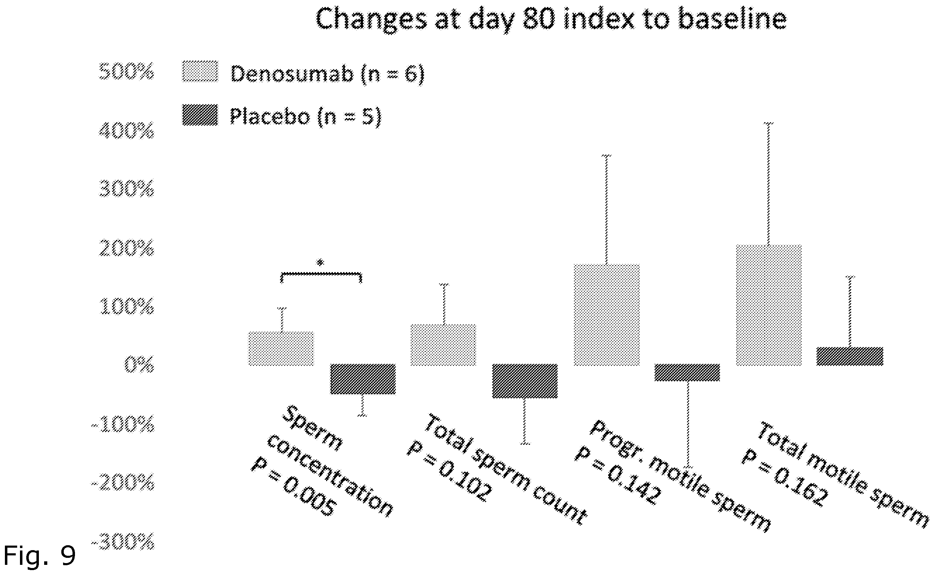

Almost all men completed the study in both arms
- Denosumab increased the fraction of spontaneous pregnancies in men with serum AMH > 30 pmol/l and AMH > 40 pmol/l compared with placebo (Figures 2 and 4).
- Denosumab increased the fraction of spontaneous pregnancies in men with sperm concentration > 15 million/ml compared with placebo (Figure 3).
- Stratification of the men into AMH tertiles showed that there was an identical beneficial response rate (semen quality improved compared with baseline) in the different AMH tertiles in the placebo treated men. While the Denosumab treated men had a higher beneficial response rate in the high AMH group and the lowest response rate in the low AMH group (Figures 5A and 5B). This was also true for total motile sperm. This indicates that AMH is a good predictive marker for the response.
- If we combine AMH with men having the highest serum RANKL/OPG ratio or highest seminal fluid then do the Denosumab treated men have a significant higher sperm output for all variables compared with placebo treated men (Figures 6-8).
- A significant increase in sperm production is seen within 80 days in men with high AMH/Inhibin B ratio and high RANKL activity defined by high serum RANKL/OPG ratio (Figure 9).
- Inhibin B in serum prior to start of treatment is a biomarker for sperm quality. (Figure 10).
- RANKL, Testosterone and Phosphate in in serum prior to start of treatment may be used as supporting biomarkers to improve the statistical value for sperm quality (Figure 11).
- AMH, Inhibin B, OPG/RANKL ratio and OPG in semen prior to start of treatment is a biomarker for sperm quality. (Figure 12).

### Conclusion

AMH level is as a strong biomarker to predict whether a male subject would benefit from a treatment to improve male fertility, especially in relation to treatment using an antagonist or inhibitor of RANKL, such as Denosumab. Inhibin B is also a strong biomarker. The predictive value can be further increased when including other biomarkers or ratio of biomarkers.

### Example 2

### Introduction

Infertility is a common problem affecting between 7-26% of all couples globally. Impaired semen quality is responsible for approximately 50% of all cases, but no treatment options to improve semen quality exist for most infertile men, except for those with hypogonadotropic hypogonadism and varicocele. The mechanisms underlying reduced sperm concentration, motility, and aberrant sperm morphology are not completely understood, but Sertoli cell function plays a central role. Spermatogenesis is strongly influenced by testosterone produced by testicular Leydig cells under the control of luteinizing hormone (LH), whereas anti-Müllerian hormone (AMH) and Inhibin B secretion from Sertoli cells are stimulated by follicle stimulating hormone (FSH). Studies in human testis and vitamin D receptor *(Vdr)* knockout mice have shown that several bone factors such as Runt-related transcription factor 2 (RUNX2), Osterix, and Osteocalcin are expressed both in normal testis as well as in testicular germ cell tumours (TGCTs). This highlights that some of these putative "endocrine bone factors" may act locally in the gonad and influence testicular function. One of these factors is the receptor activator of NF-,cB ligand (RANKL).

The RANKL system is a powerful regulator of bone resorption that comprises three components: RANKL, a transmembrane ligand that upon binding to its receptor RANK on a neighbouring cell activates NF-κB and regulates cell cycle i.e. proliferation, differentiation, and apoptosis. The transmembrane RANKL resides in bone-forming cells and activates RANK in immature osteoclasts and thereby induces osteoclastogenesis and promotes bone resorption. Osteoprotegerin (OPG) is a secreted decoy receptor that binds RANKL and blocks RANK activation and prevents osteoclast differentiation and activation. RANKL can also be found in serum, suggesting a putative endocrine function for example in the regulation of glucose homeostasis. Moreover, a recent study suggested that reverse RANKL signaling occurs when soluble RANK binds to RANKL and induces signaling. Here, we show that RANKL, RANK, and OPG are expressed in different cell types in the testis, suggesting a yet unrecognized regulatory role of spermatogenesis. These findings may ultimately be of clinical relevance since an FDA/EMA approved specific RANKL inhibitor, Denosumab, is in use to treat osteoporosis in women and men. OPG was used clinically initially but discontinued due to off target inhibition of tumour necrosis factor-related apoptosis-inducing ligand (TRAIL) and a short half-life. Denosumab was developed to avoid off target effects and has a longer half-life, which implied that patients only require injections every six months for treatment of osteoporosis. Here, we show that bone, the immune system, and the male gonads share the RANKL/RANK/OPG signalling system; and by using exogenous and genetic repression of RANKL systemically and specifically in Sertoli cells in mice as well as descriptive and functional human studies we provide insights into the relevance of RANKL as a modifiable regulator of male reproductive function.

### Materials and methods

TNFSF11 Inhibition and Fertility: a Prospective intervention Study NCT02422108: To address the relevance of RANKL inhibition on human male reproduction, Denosumab (Prolia) 60 mg was injected into 12 infertile men once and semen quality was monitored for 180 days (see also example 1 above).

The study was approved by the local ethics committee H-15001992 and was conducted and monitored according to GCP standard. All 12 infertile men had impaired semen quality and the severity varied from mild to severe oligospermia. Their bone health evaluated by DXA showed normal BMD and all had normal serum calcium, phosphate, alkaline phosphatase, 25-OHD and PTH levels at baseline. All men delivered 2 semen samples prior to treatment start and serum RANKL and OPG were measured concomitantly with reproductive and calciotropic hormones at baseline (S-table). In total 22 men were screened, and 12 men were included. All men received calcium and vitamin D supplementation prior to treatment. After treatment start, all 12 men were requested to deliver semen and blood samples at day 5, 20, 40, 80, 120, and 180. The "early time points" were included to avoid missing a putative antiapoptotic effect due to the fast response of RANKL inhibition observed in human *ex vivo* models and wild-type mice.

### Biochemical analysis:

Fasting serum samples and seminal fluid were analyzed at all timepoints using validated methodology. Fasting blood samples were collected between 8:00 and 10:00 AM. Serum was analyzed immediately for calcium (total and ionized), phosphate and PTH. The remaining analyses were conducted on thawed serum samples.

### Semen analysis:

Semen samples were delivered in an adjacent room in the out-patient clinic and information on duration of abstinence, fever, and spillage was obtained. The two semen samples were delivered 10-16 days apart prior to treatment start and analysis was conducted exactly as described in detail previously. Briefly, semen volume was determined by weighing, sperm concentration was determined using a Bürker-Türk hemocytometer, and total sperm count was calculated. Sperm morphology was assessed according to strict criteria on Papanicolaou-stained smears. Sperm motility classified as progressive motile (WHO class A+B), nonprogressive motile (class C), or immotile (class D) was determined in duplicate at two times and presented as AB or ABC motility. Spermatozoa DNA fragmentation was investigated at baseline and 20 and 120 days after intervention at SPZ Laboratory (Copenhagen, Denmark). In brief, 0.5 mL semen was diluted with TNE buffer, then mixed and frozen directly in liquid nitrogen until fluorescent staining according to the sperm chromatin structure assay protocol and then analyzed using a FACSCalibur (BD Biosciences, San Jose,CA) flow cytometer. All samples were run blinded in duplicate and recording stopped after 5000 events.

### Statistics:

5-18 male mice at 16-17 weeks of age were used from each genotype to reach statistical significance - this number was calculated (power calculation) based on previous experience. All analyses were done by technicians or researchers that were blinded to genotype and/or treatment. All values were expressed as mean ± SEM. p<0.05 was considered statistically significant. The following phenotypic variables: sperm count, organ weight, motility and gene-expression (some logarithmic transformed) were tested using Student's t-test or one-way analysis of variance (ANOVA) with Dunnett's test to adjust for multiple comparisons. No outliers were excluded from analyses. Cross-sectional data from Copenhagen bone gonadal study were expressed as mean ± SEM except for sperm concentration and total sperm count (Median). Associations between serum or seminal fluid levels of RANKL and OPG with semen quality and reproductive hormones were conducted after natural logarithmic transformation and adjustment for BMI, age, and duration of abstinence for all semen variables. Subsequently, all men were stratified according to WHO criteria for semen quality variables. Evaluation of gaussian distribution was done by plotting residuals and as a result the following variables were transformed with natural logarithm: RANKL in seminal fluid, RANKL in serum, seminal/serum RANKL ratio, total number of sperm, semen concentration, concentration of progressive motile sperm, and concentration of morphological normal sperm. Seminal RANKL levels were set as dependent in linear regression analyses with hormones, whereas it was set independent in linear regression with seminal parameters. RANKL levels, hormonal levels, and seminal parameters in infertile men were compared with levels in in infertile men and healthy men followed by a pooled analysis of all the men. All hormonal analyses were adjusted for BMI and analyses on semen quality were also adjusted for duration of abstinence.

### TNFSF11 Inhibition and Fertility:

The primary endpoint was changes in sperm production from day 80 and onwards compared with baseline. The following predefined secondary endpoints were also evaluated: Sperm DFI, FSH, Inhibin B, AMH, serum OPG, and sRANKL. Based on the prespecified statistical analyses each timepoint was compared with baseline by paired t-test starting with day 80. Subjects who terminated participation after visit day 1 but before visit day 180 were included for data analysis. Men with fever above 38.5°C were included in the analyses 3 months after the fever episode. Spermatogenesis takes 74 days in men and the initial investigation was to determine the difference to all timepoints and calculate average for days 80, 120, and 180 and compare with baseline values to determine the effect of RANKL inhibition after a full length of spermatogenesis. Subsequent analyses were conducted after stratification into predefined subgroups subjects according to BMI, serum RANKL and OPG before treatment.

### Results

RANKL, RANK, and OPG are expressed in the human testis and regulate germ cell apoptosis.

A marked transcriptional expression of *RANKL, RANK,* and *OPG* was found in testicular tissue (data not shown). All three isoforms of RANKL were expressed in the human testis including the transmembrane and soluble form of the protein. By using antibodies targeting the transmembrane or extracellular domain we found expression of RANKL in the SOX9 positive Sertoli cells and some of the germ cells on the luminal site of the blood-testis barrier. The level of RANKL expression varied between samples but was detected primarily in the cytoplasm/membrane of the Sertoli cells and some spermatocytes and spermatids but not in spermatogonia. RANK was expressed in the cytoplasm/membrane of the germ cells with the most prominent expression in spermatogonia, while OPG was expressed mainly in the spermatogonia and less prominently in peritubular cells or on the border between the peritubular cells and spermatogonia and in some spermatids. The soluble isoform of RANKL (sRANKL, observed at 27-31 kDa) and the transmembrane isoform (45 kDa) were both expressed in the testis. Both isoforms of RANKL, RANK, and OPG were robustly expressed in samples with normal spermatogenesis. Human testicular tissue was cultured *ex vivo* to preserve the functional integrity and to support continued cell-cell signalling in order to investigate effects of RANKL-inhibition on germ cell apoptosis. Most of the included specimens contained tubules with spermatogenic arrest or some atrophy. Despite the low number of tubules with complete spermatogenesis, treatment with Denosumab or OPG for 48 hours reduced the number of apoptotic cells (assessed by number of cleaved PARP⁺ cells per area) by 30% compared with vehicle control (p<0.05). Denosumab or OPG treatment did not significantly affect germ cell proliferation (determined by number of BrdU⁺ cells per area), although a tendency towards an increase was observed Interestingly, the release of sRANKL into the culture media was significantly reduced by Denosumab or OPG treatment compared with vehicle treatment (p<0.05).

Denosumab suppress RANKL levels in seminal fluid and increase circulating Inhibin B in infertile men.

To test efficacy and safety of RANKL inhibition in infertile men, a single dose of Denosumab (Prolia^{®}, 60 mg) was injected subcutaneously into 12 infertile men. Semen quality, reproductive hormones, calcium homeostasis, sRANKL, and OPG were evaluated at day 5, 20, 40, 80, 120, and 180 after injection to avoid missing an early antiapoptotic effect (day 5, 20, 40), but most importantly to assess semen quality after a full cycle of spermatogenesis (74 days) on day 80. Therefore, the prespecified statistical analysis stated that direct comparisons between timepoints starting at day 80 with baseline were to be conducted initially to avoid adjusting for multiple adjustments. Semen quality variables and hormones were normalized to the average of the two samples delivered prior to treatment start. One man was lost to follow up and one man had high fever at the beginning of the trial and was therefore per protocol excluded from the analysis leaving 10 men in the study.

The results are explained in detail below.

Serum levels of AMH and Inhibin B were significantly higher at day 80 (p=0.003 and p=0.018) compared with baseline. Inhibin B levels increased in all men except for one when comparing baseline with day 80. Serum levels of FSH did not change, but the Inhibin B/FSH ratio increased significantly at day 80 (p=0.02). Serum estradiol concentration was significantly lower at day 180 (p=0.001), while testosterone, SHBG, and LH concentrations remained unchanged. Total sperm count, sperm concentration, sperm motility, and total number of progressive motile sperm were not higher at day 80. However, at day 80 the number of progressive motile spermatozoa (p=0.057) and motile spermatozoa (p=0.071) tended to be higher compared with baseline. Interestingly, in a sub-group of the men (60%) an increase in the number of progressive motile sperm (between 100-500%) was found when compared with their baseline. Surprisingly, the remaining 40% of men experienced a marked decrease in number of progressive motile spermatozoa. This preliminary finding could indicate that the effect of Denosumab is dependent on specific baseline characteristics of the patients. Assessment of clinical and biochemical differences at baseline indicated that high serum AMH, Inhibin B, AMH/Inhibin B, RANKL/OPG ratio prior to treatment were the best predictor for a positive treatment response to Denosumab. Men with OPG levels >3.0 pmol/L had a significant increase in total motile sperm and total progressive motile sperm after Denosumab treatment at day 80 (both, p<0.05). Serum Inhibin B and AMH was also good predictors, while serum RANKL, 1,25(OH)₂D₃, Klotho, and phosphate were the best negative predictors for the Denosumab-induced increase in total progressive motile sperm or Inhibin B. Men with high OPG or high calculated RANKL also had higher Inhibin B and AMH levels. Noteworthy, sRANKL concentration in serum was completely repressed instantly in all men following injection of Denosumab and was detectable again (except in one man) at day 120 or 180. In contrast, OPG increased at day 20 and 40 after denosumab injection before returning to baseline levels. Furthermore, Denosumab lowered sRANKL levels in the seminal fluid, but seminal sRANKL concentration was not completely repressed and remained detectable and higher than serum levels in most men at day 80 and 120. Noteworthy, 2 out of the 10 men who enrolled and completed the study were responsible for a pregnancy during the study and their spouses conceived naturally with two healthy live births.

### Discussion

The present example demonstrates presence of the RANKL-system in the male reproductive organs of both mice and humans. Expression of RANKL in Sertoli cells, spermatocytes, and spermatids, the receptor RANK in spermatogonia and spermatids, and the inhibitor OPG in spermatogonia and peritubular cells highlights the dependence of intimate cell-cell interaction as has been shown for RANKL-signaling in bone. Importantly, the testicular expression pattern of RANKL-signaling is conserved between mice and humans and *ex vivo* cultures of human testicular tissue allowed us to compare the effects of OPG and Denosumab treatment on human germ cell apoptosis and proliferation. The comparable antiapoptotic effect of OPG and Denosumab on human germ cells and suppression of RANKL released to the media supports the notion that the stimulatory effect is mediated by RANKL signaling rather than through TRAIL. Additionally, Denosumab and OPG did not significantly increase the number of proliferating germ cells in *ex vivo* cultures, suggesting that the effects on sperm counts were most likely the result of reduced germ cell apoptosis.

The 100-fold higher sRANKL concentration in seminal fluid compared with serum supports a biological role and it is plausible that RANKL is secreted, cleaved, or released from the epithelial cells in the reproductive tract as there was no link between serum and seminal concentrations of sRANKL. Moreover, the strong associations with serum testosterone and estradiol suggest that RANKL activity and release in the epididymis and prostate may be influenced by sex steroids. A biological role of seminal RANKL was indicated by the negative associations between seminal sRANKL concentrations and all semen quality variables including the total number of progressive motile sperm and total number of morphological normal sperm that refers to the number of mature and functional sperm and not just sperm quantity. The associations between semen quality variables and seminal RANKL levels were not strong, which implies that other regulators such as FSH and testosterone likely exert a stronger regulatory effect on spermatogenesis and sperm maturation than seminal RANKL concentration. Stratification of men according to WHO references for normal semen quality revealed that men with poor semen quality, including low number of sperm, low motility, or morphologically abnormal sperm had higher seminal sRANKL than men with normal semen quality. The seminal/serum RANKL ratio had an even higher AUC and was a better marker than all semen quality variables except for sperm motility. No causal evidence for a direct negative effect of RANKL in the seminal fluid has been found and it is questionable whether Denosumab could enter the luminal site of testis or epididymis and reduce seminal RANKL levels due to the blood-testis and blood-epididymis barrier.

We conducted a small intervention trial using a standard osteoporosis dose of Denosumab (60 mg, once) in infertile men. This caused an immediate suppression of serum sRANKL while OPG remained high. Interestingly, sRANKL concentration in seminal fluid was reduced but not fully repressed following treatment with the standard dosage regime for osteoporosis. It is unknown whether a more potent repression of seminal fluid sRANKL concentration may lead to an increased treatment response since it is likely that the optimal doses required for osteoporosis and improvement of male reproductive function may differ. Analysis of multiple timepoints were included in this trial to avoid missing an early antiapoptotic effect, but the prespecified statistical analysis states that we should initially compare endpoints after one length of spermatogenesis (74 days) with baseline to avoid adjustment for multiple comparisons. Denosumab induced an increase in serum AMH and Inhibin B after 80. Subgroup analysis showed that in infertile men with high serum AMH, Inhibin B, RANKL and OPG levels, Denosumab increased the number of total progressive motile sperm, which supports a regulatory role of RANKL in male reproduction. Seminal fluid sRANKL concentrations were positively associated with serum estradiol and negatively with serum testosterone. This observation is of great interest as infertile men tend to have lower testosterone/estradiol ratio than normal men, which may facilitate the high seminal sRANKL levels and is in accordance with the regulatory role of sex steroids on RANKL and OPG in the skeleton. Noteworthy, Denosumab treatment in infertile men had no effect on serum gonadotropin or testosterone concentrations but there was a late increase in serum estradiol.

Furthermore, almost all infertile men had an increase in AMH and Inhibin B levels, but only 60% of them were able to convert these endocrine changes into an increased number of progressive motile sperm. We cannot explain this discrepancy, but it could be due to heterogenicity in the etiology of their infertility. AMH and Inhibin B may be the best positive predictive markers, which indicates that infertile men with the best residual testicular function will be more likely to benefit from the treatment.

### Conclusion

This study suggests that RANKL signaling is a novel regulator of male reproductive function by mediating interactions between Sertoli-germ-peritubular cells within the testis and in the male reproductive tract, to influence semen quality and male fertility.

## Claims

1. A method for determining a likely effect of a treatment to improve male fertility, the method comprising
• determining the level of AMH and/or inhibin B in a biological sample from a male subject;
• comparing said determined AMH and/or inhibin B level to a corresponding reference level; and
• determining that said subject will
∘ likely benefit from treatment with an antagonist or inhibitor of RANKL, if said level is equal to or higher than said reference level; or
∘ likely not benefit from treatment with an antagonist or inhibitor of RANKL, if said level is lower than said reference level.

2. The method according to claim 1, wherein at least the level of AMH is determined.

3. The method according to claim 1 or 2, wherein said reference level is
• above 30 pmol/l AMH in blood serum, such as at least 35 pmol/l AMH or such as at least 40 pmol/l AMH; and/or
• above 5 pmol/l AMH in seminal fluid.

4. The method according to any of the preceding claims, wherein
• said reference level in blood serum is in the range 30 to 100 pmol/l AMH, such as in the range 30 to 80 pmol/l AMH; and/or
• said reference level in seminal fluid is in the range in the range 5 pmol/l AMH up to 1000 pmol/L.

5. The method according to any of the preceding claims, wherein the antagonist or inhibitor of RANKL having binding affinity for RANKL, binds RANKL at the same epitope as Denosumab or OPG, and/or binds RANKL with a binding affinity similar to Denosumab or OPG or with a binding affinity higher than Denosumab or OPG.

6. The method according to any of the preceding claims, wherein said inhibitor of RANKL is selected from the group consisting of Denosumab or fragments thereof (able to bind to RANKL), OPG or fragments thereof (able to bind to RANKL).

7. The method according to any of the preceding claims, further comprising
• determining the level of OPG and/or Inhibin B, and/or RANKL and/or RANKL/OPG ratio and/or AMH/inhibin B ratio and/or AMH/testosterone ratio in a biological sample from the male subject;
• comparing said determined level of OPG and/or Inhibin B, and/or RANKL and/or RANKL/OPG ratio and/or AMH/inhibin B ratio and/or AMH/testosterone ratio to a corresponding reference level; and
• determining that said subject will
∘ likely benefit from treatment with an antagonist or inhibitor of RANKL, if said one or more levels are equal to or higher than said reference level; or
∘ likely not benefit from treatment with an antagonist or inhibitor of RANKL, if said on or more levels are lower than said reference level.

8. The method according to claim 7, wherein
• said reference level in blood serum is at least 50 IU/ml Inhibin B, such as at least 100 IU/ml Inhibin B or such as at least 200 IU/ml Inhibin B, or such as in the range 50 to 500 IU/ml Inhibin B, or such as in the range 200 to 500 IU/ml Inhibin B; and/or
• said reference level in seminal fluid is at least 20 IU/ml Inhibin B, such as at least 100 IU/ml Inhibin B or such as at least 200 IU/ml Inhibin B, or such as in the range 20 to 500 IU/ml Inhibin B, or such as in the range 20 to 10000 IU/ml Inhibin B;
and/or
• said reference level in blood serum is in the range 2 to 5 pmol/l OPG, such as in the range 3-5 pmol/l OPG or such as in the range 3-4 pmol/l OPG; and/or
• said reference level in seminal fluid is in the range in the range 5 to 50 pmol/l OPG, such as in the range 10 to 40 pmol/l OPG;
and/or
• said reference level in blood serum is in the range 0.1 to 0.5 pmol/l soluble RANKL, such as in the range 0.2-0.44 pmol/l RANKL; and/or
• said reference level in seminal fluid is in the range 1-76 pmol/L seminal sRANKL, such as 20-76 pmol/l seminal sRANKL, 40-76 pmol/l seminal sRANKL, 1-50 pmol/l seminal sRANKL, 1-30 pmol/l seminal sRANKL, or 1-20 pmol/l seminal sRANKL.
and/or
• said reference level in blood serum is an OPG/RANKL below 30, such as below 20, or such as below 15; and/or
• said reference level in blood seminal fluid is an OPG/RANKL below 30, such as below 20, or such as below 15.

9. The method according to any of the preceding claims, further comprising
• determining the level of testosterone and/or phosphate in a biological sample from the male subject;
• comparing said determined level of testosterone and/or phosphate to a corresponding reference level; and
• determining that said subject will
∘ likely benefit from treatment with an antagonist or inhibitor of RANKL, if said one or more levels are equal to or higher than said reference level; or
∘ likely not benefit from treatment with an antagonist or inhibitor of RANKL, if said on or more levels are lower than said reference level.

10. The method according to claim 9, wherein the reference level for serum testosterone is > 10 nmol/l, such as in the range 10-40 nmol/l, and/or wherein the reference level for serum phosphate is > 0.6 mmol/l, such as in the range 0.6-1.0 mmol/l.

11. The method according to any of the preceding claims, wherein said subject suffers from oligospermia.

12. Use of AMH and/or inhibin B as a biomarker for determining a likely effect of a treatment to improve male fertility, wherein the treatment is with an antagonist or inhibitor of RANKL.

13. The use according to claim 13, wherein a level above 30 pmol/l AMH in blood serum is indicative of a likely positive effect on male infertility of the treatment is with an antagonist or inhibitor of RANKL and/or a level above 5 pmol/l AMH in seminal fluid is indicative of a likely positive effect on male infertility of the treatment is with an antagonist or inhibitor of RANKL

14. A composition comprising an antagonist or inhibitor of RANKL for use in the treatment of male infertility in subject, wherein said subject has a level of AMH of:
• above 30 pmol/l in blood serum, such as at least 35 pmol/l AMH or such as at least 40 pmol/l AMH; and/or
• above 5 pmol/l AMH in seminal fluid.

## Patentansprüche

1. Verfahren zur Bestimmung einer wahrscheinlichen Wirkung einer Behandlung zur Verbesserung der männlichen Fruchtbarkeit, wobei das Verfahren Folgendes umfasst
• Bestimmen des AMH- und/oder Inhibin B-Spiegels in einer biologischen Probe von einem männlichen Subjekt;
• Vergleichen des bestimmten AMH- und/oder Inhibin B-Spiegels mit einem entsprechenden Referenzspiegel; und
• Bestimmen, dass das Subjekt
∘ wahrscheinlich von einer Behandlung mit einem Antagonisten oder Inhibitor von RANKL profitieren wird, wenn der Spiegel gleich oder höher als der Referenzspiegel ist; oder
∘ wahrscheinlich nicht von einer Behandlung mit einem Antagonisten oder Inhibitor von RANKL profitieren wird, wenn der Spiegel niedriger als der Referenzspiegel ist.

2. Verfahren nach Anspruch 1, wobei zumindest der AMH-Spiegel bestimmt wird.

3. Verfahren nach Anspruch 1 oder 2, wobei der Referenzspiegel
• oberhalb von 30 pmol/l AMH im Blutserum, wie zum Beispiel mindestens 35 pmol/l AMH oder wie zum Beispiel mindestens 40 pmol/l AMH; und/oder
• oberhalb von 5 pmol/l AMH in der Samenflüssigkeit ist.

4. Verfahren nach einem der vorstehenden Ansprüche, wobei
• der Referenzspiegel im Blutserum im Bereich von 30 bis 100 pmol/l AMH liegt, wie zum Beispiel im Bereich von 30 bis 80 pmol/l AMH; und/oder
• der Referenzspiegel in der Samenflüssigkeit im Bereich von 5 pmol/l AMH bis zu 1000 pmol/l liegt.

5. Verfahren nach einem der vorstehenden Ansprüche, wobei der Antagonist oder Inhibitor von RANKL mit einer Bindungsaffinität für RANKL dieses an dem gleichen Epitop wie Denosumab oder OPG bindet und/oder RANKL mit einer ähnlichen Bindungsaffinität wie Denosumab oder OPG oder mit einer höheren Bindungsaffinität als Denosumab oder OPG bindet.

6. Verfahren nach einem der vorstehenden Ansprüche, wobei der Inhibitor von RANKL ausgewählt ist aus der Gruppe bestehend aus Denosumab oder Fragmenten davon (die an RANKL binden können), OPG oder Fragmenten davon (die an RANKL binden können).

7. Verfahren nach einem der vorstehenden Ansprüche, weiter umfassend
• Bestimmen des Spiegels von OPG und/oder Inhibin B und/oder von RANKL und/oder des RANKL/OPG-Verhältnisses und/oder des AMH/Inhibin B-Verhältnisses und/oder des AMH/Testosteron-Verhältnisses in einer biologischen Probe von dem männlichen Subjekt;
• Vergleichen des bestimmten Spiegels von OPG und/oder Inhibin B und/oder von RANKL und/oder des RANKL/OPG-Verhältnisses und/oder AMH/Inhibin B-Verhältnisses und/oder AMH/Testosteron-Verhältnisses mit einem entsprechenden Referenzspiegel; und
• Bestimmen, dass das Subjekt
∘ wahrscheinlich von einer Behandlung mit einem Antagonisten oder Inhibitor von RANKL profitieren wird, wenn der eine oder die mehreren Spiegel gleich oder höher als der Referenzspiegel sind; oder
∘ wahrscheinlich nicht von einer Behandlung mit einem Antagonisten oder Inhibitor von RANKL profitieren wird, wenn der eine oder die mehreren Spiegel niedriger als der Referenzspiegel sind.

8. Verfahren nach Anspruch 7, wobei
• der Referenzspiegel im Blutserum mindestens 50 IE/ml Inhibin B, wie zum Beispiel mindestens 100 IE/ml Inhibin B oder wie zum Beispiel mindestens 200 IE/ml Inhibin B, oder wie zum Beispiel im Bereich von 50 bis 500 IE/ml Inhibin B, oder wie zum Beispiel im Bereich von 200 bis 500 IE/ml Inhibin B liegt; und/oder
• der Referenzspiegel in der Samenflüssigkeit mindestens 20 IE/ml Inhibin B, wie zum Beispiel mindestens 100 IE/ml Inhibin B oder wie zum Beispiel mindestens 200 IE/ml Inhibin B, oder wie zum Beispiel im Bereich von 20 bis 500 IE/ml Inhibin B, oder wie zum Beispiel im Bereich von 20 bis 10000 IE/ml Inhibin B liegt;
und/oder
• der Referenzspiegel im Blutserum im Bereich von 2 bis 5 pmol/l OPG liegt, wie zum Beispiel im Bereich von 3-5 pmol/l OPG oder wie zum Beispiel im Bereich von 3-4 pmol/l OPG; und/oder
• der Referenzspiegel in der Samenflüssigkeit im Bereich von 5 bis 50 pmol/l OPG liegt, wie zum Beispiel im Bereich von 10 bis 40 pmol/l OPG;
und/oder
• der Referenzspiegel im Blutserum im Bereich von 0,1 bis 0,5 pmol/l löslichem RANKL liegt, wie zum Beispiel im Bereich von 0,2-0,44 pmol/l RANKL; und/oder
• der Referenzspiegel in der Samenflüssigkeit im Bereich von 1-76 pmol/l seminalem sRANKL liegt, wie zum Beispiel 20-76 pmol/l seminalem sRANKL, 40-76 pmol/l seminalem sRANKL, 1-50 pmol/l seminalem sRANKL, 1-30 pmol/l seminalem sRANKL oder 1-20 pmol/l seminalem sRANKL.
und/oder
• der Referenzspiegel im Blutserum ein OPG/RANKL-Wert unter 30, wie zum Beispiel unter 20 oder wie zum Beispiel unter 15 ist; und/oder
• der Referenzspiegel in der Samenflüssigkeit ein OPG/RANKL-Wert unter 30, wie zum Beispiel unter 20 oder wie zum Beispiel unter 15 ist.

9. Verfahren nach einem der vorstehenden Ansprüche, weiter umfassend
• Bestimmen des Spiegels von Testosteron und/oder Phosphat in einer biologischen Probe von dem männlichen Subjekt;
• Vergleichen des bestimmten Testosteron- und/oder Phosphatspiegels mit einem entsprechenden Referenzspiegel; und
• Bestimmen, dass das Subjekt
∘ wahrscheinlich von einer Behandlung mit einem Antagonisten oder Inhibitor von RANKL profitieren wird, wenn der eine oder die mehreren Spiegel gleich oder höher als der Referenzspiegel sind; oder
∘ wahrscheinlich nicht von einer Behandlung mit einem Antagonisten oder Inhibitor von RANKL profitieren wird, wenn der eine oder die mehreren Spiegel niedriger als der Referenzspiegel sind.

10. Verfahren nach Anspruch 9, wobei der Referenzspiegel für Serumtestosteron > 10 nmol/l ist, wie zum Beispiel im Bereich von 10-40 nmol/l, und/oder wobei der Referenzspiegel für Serumphosphat > 0,6 mmol/l ist, wie zum Beispiel im Bereich von 0,6-1,0 mmol/l.

11. Verfahren nach einem der vorstehenden Ansprüche, wobei das Subjekt an Oligospermie leidet.

12. Verwendung von AMH und/oder Inhibin B als Biomarker zur Bestimmung einer wahrscheinlichen Wirkung einer Behandlung zur Verbesserung der männlichen Fruchtbarkeit, wobei die Behandlung mit einem Antagonisten oder Inhibitor von RANKL erfolgt.

13. Verwendung nach Anspruch 13, wobei ein AMH-Spiegel von über 30 pmol/l im Blutserum auf einen wahrscheinlichen positiven Effekt der Behandlung mit einem Antagonisten oder Inhibitor von RANKL auf die männliche Unfruchtbarkeit hinweist und/oder ein Spiegel oberhalb von 5 pmol/l AMH in der Samenflüssigkeit auf einen wahrscheinlichen positiven Effekt der Behandlung mit einem Antagonisten oder Inhibitor von RANKL auf die männliche Unfruchtbarkeit hinweist.

14. Zusammensetzung, die einen Antagonisten oder Inhibitor von RANKL umfasst, zur Verwendung bei der Behandlung von männlicher Unfruchtbarkeit bei einem Subjekt, wobei das Subjekt einen AMH-Spiegel von
• oberhalb von 30 pmol/l im Blutserum, wie zum Beispiel mindestens 35 pmol/l AMH oder wie zum Beispiel mindestens 40 pmol/l AMH; und/oder
• über 5 pmol/l AMH in der Samenflüssigkeit aufweist.

## Revendications

1. Procédé pour déterminer un effet probable d'un traitement destiné à améliorer la fertilité masculine, le procédé comprenant les étapes consistant à
• déterminer le taux d'AMH et/ou d'inhibine B dans un échantillon biologique provenant d'un sujet masculin ;
• comparer ledit taux d'AMH et/ou d'inhibine B déterminé à un taux de référence correspondant ; et
• déterminer que ledit sujet
∘ bénéficiera probablement d'un traitement par un antagoniste ou inhibiteur de RANKL, si ledit taux est égal ou supérieur audit taux de référence ; ou
∘ ne bénéficiera probablement pas d'un traitement par un antagoniste ou inhibiteur de RANKL, si ledit taux est inférieur audit taux de référence.

2. Procédé selon la revendication 1, dans lequel au moins le taux d'AMH est déterminé.

3. Procédé selon la revendication 1 ou 2, dans lequel ledit taux de référence est
• supérieur à 30 pmoles/l d'AMH dans le sérum sanguin, tel que d'au moins 35 pmoles/l d'AMH ou tel que d'au moins 40 pmoles/l d'AMH ; et/ou
• supérieur à 5 pmoles/l d'AMH dans le liquide séminal.

4. Procédé selon l'une quelconque des revendications précédentes, dans lequel
• ledit taux de référence dans le sérum sanguin est dans la plage de 30 à 100 pmoles/l d'AMH, tel que dans la plage de 30 à 80 pmoles/l d'AMH ; et/ou
• ledit taux de référence dans le liquide séminal est dans la plage de 5 pmoles/l d'AMH à 1 000 pmoles/L.

5. Procédé selon l'une quelconque des revendications précédentes, dans lequel l'antagoniste ou inhibiteur de RANKL, ayant une affinité de liaison pour le RANKL, se lie au RANKL au niveau du même épitope que le dénosumab ou l'OPG, et/ou se lie au RANKL avec une affinité de liaison semblable à celle du dénosumab ou de l'OPG ou avec une affinité de liaison supérieure à celle du dénosumab ou de l'OPG.

6. Procédé selon l'une quelconque des revendications précédentes, dans lequel ledit inhibiteur de RANKL est choisi dans le groupe constitué par le dénosumab ou des fragments de celui-ci (capables de se lier au RANKL), l'OPG ou des fragments de celle-ci (capables de se lier au RANKL).

7. Procédé selon l'une quelconque des revendications précédentes, comprenant en outre les étapes consistant à
• déterminer le taux d'OPG et/ou d'inhibine B, et/ou de RANKL et/ou le rapport RANKL/OPG et/ou le rapport AMH/inhibine B et/ou le rapport AMH/testostérone dans un échantillon biologique provenant du sujet masculin ;
• comparer ledit taux d'OPG et/ou d'inhibine B, et/ou de RANKL et/ou rapport RANKL/OPG et/ou rapport AMH/inhibine B et/ou rapport AMH/testostérone déterminé à un taux de référence correspondant ; et
• déterminer que ledit sujet
∘ bénéficiera probablement d'un traitement par un antagoniste ou inhibiteur de RANKL, si lesdits un ou plusieurs taux sont égaux ou supérieurs audit taux de référence ; ou
∘ ne bénéficiera probablement pas d'un traitement par un antagoniste ou inhibiteur de RANKL, si lesdits un ou plusieurs taux sont inférieurs audit taux de référence.

8. Procédé selon la revendication 7, dans lequel
• ledit taux de référence dans le sérum sanguin est d'au moins 50 UI/ml d'inhibine B, tel que d'au moins 100 UI/ml d'inhibine B ou tel que d'au moins 200 UI/ml d'inhibine B, ou tel que dans la plage de 50 à 500 UI/ml d'inhibine B, ou tel que dans la plage de 200 à 500 UI/ml d'inhibine B ; et/ou
• ledit taux de référence dans le liquide séminal est d'au moins 20 UI/ml d'inhibine B, tel que d'au moins 100 UI/ml d'inhibine B ou tel que d'au moins 200 UI/ml d'inhibine B, ou tel que dans la plage de 20 à 500 UI/ml d'inhibine B, ou tel que dans la plage de 20 à 10 000 UI/ml d'inhibine B ;
et/ou
• ledit taux de référence dans le sérum sanguin est dans la plage de 2 à 5 pmoles/l d'OPG, tel que dans la plage de 3-5 pmoles/l d'OPG ou tel que dans la plage de 3-4 pmoles/l d'OPG ; et/ou
• ledit taux de référence dans le liquide séminal est dans la plage de 5 à 50 pmoles/l d'OPG, tel que dans la plage de 10 à 40 pmoles/l d'OPG ;
et/ou
• ledit taux de référence dans le sérum sanguin est dans la plage de 0,1 to 0,5 pmoles/l de RANKL soluble, tel que dans la plage de 0,2-0,44 pmoles/l de RANKL ; et/ou
• ledit taux de référence dans le liquide séminal est dans la plage de 1-76 pmoles/L de sRANKL séminal, tel que de 20-76 pmoles/l de sRANKL séminal, 40-76 pmoles/l de sRANKL séminal, 1-50 pmoles/l de sRANKL séminal, 1-30 pmoles/l de sRANKL séminal, ou 1-20 pmoles/l de sRANKL séminal ;
et/ou
• ledit taux de référence dans le sérum sanguin est un rapport OPG/RANKL inférieur à 30, tel qu'inférieur à 20, ou tel qu'inférieur à 15 ; et/ou
• ledit taux de référence dans le liquide séminal est un rapport OPG/RANKL inférieur à 30, tel qu'inférieur à 20, ou tel qu'inférieur à 15.

9. Procédé selon l'une quelconque des revendications précédentes, comprenant en outre les étapes consistant à
• déterminer le taux de testostérone et/ou phosphate dans un échantillon biologique provenant du sujet masculin ;
• comparer ledit taux de testostérone et/ou phosphate déterminé à un taux de référence correspondant ; et
• déterminer que ledit sujet
∘ bénéficiera probablement d'un traitement par un antagoniste ou inhibiteur de RANKL, si lesdits un ou plusieurs taux sont égaux ou supérieurs audit taux de référence ; ou
∘ ne bénéficiera probablement pas d'un traitement par un antagoniste ou inhibiteur de RANKL, si lesdits un ou plusieurs taux sont inférieurs audit taux de référence.

10. Procédé selon la revendication 9, dans lequel le taux de référence pour la testostérone sérique est > 10 nmoles/l, tel que dans la plage de 10-40 nmoles/l, et/ou dans lequel le taux de référence pour le phosphate sérique est > 0,6 mmole/l, tel que dans la plage de 0,6-1,0 mmole/l.

11. Procédé selon l'une quelconque des revendications précédentes, dans lequel ledit sujet souffre d'oligospermie.

12. Utilisation d'AMH et/ou d'inhibine B en tant que biomarqueur pour déterminer un effet probable d'un traitement pour améliorer la fertilité masculine, dans laquelle le traitement est par un antagoniste ou inhibiteur de RANKL.

13. Utilisation selon la revendication 13, dans laquelle un taux supérieur à 30 pmoles/l d'AMH dans le sérum sanguin est indicateur d'un effet positif probable sur l'infertilité masculine du traitement par un antagoniste ou inhibiteur de RANKL et/ou un taux supérieur à 5 pmoles/l d'AMH dans le liquide séminal est indicateur d'un effet positif probable sur l'infertilité masculine du traitement par un antagoniste ou inhibiteur de RANKL.

14. Composition comprenant un antagoniste ou inhibiteur de RANKL destinée à être utilisée dans le traitement de l'infertilité masculine chez un sujet, dans laquelle ledit sujet à un taux d'AMH de :
• plus de 30 pmoles/l dans le sérum sanguin, tel que d'au moins 35 pmoles/l d'AMH ou tel que d'au moins 40 pmoles/l d'AMH ; et/ou
• plus de 5 pmoles/l d'AMH dans le liquide séminal.
